# EUROPÄISCHE PATENTSCHRIFT

(11) **EP 4 065 561 B1**
(45) Veröffentlichungstag und Bekanntmachung des Hinweises auf die Patenterteilung: **16.08.2023**
(21) Anmeldenummer: 20726866.5
(22) Anmeldetag: 26.05.2020
(51) Int. Cl.: C07D 211/94

(54) **VERFAHREN ZUR HERSTELLUNG SALZARMER WÄSSRIGER LÖSUNGEN VON 4-AMMONIUM-ALKYLPIPERIDIN-1-YLOXYLSALZEN ZUM EINSATZ IN LADUNGSSPEICHEREINHEITEN**
PROCESS FOR THE PREPARATION OF AQUEOUS SOLUTIONS OF LOW SALT CONCENTRATION OF 4-AMMONIUM-ALKYLPIPERIDIN-1-YLOXY SALTS FOR USE IN CHARGE-STORAGE UNITS
PROCÉDÉ DE PRÉPARATION DE SOLUTIONS AQUEUSES À FAIBLE TENEUR EN SEL DE SELS 4-AMMONIUM-ALKYLPIPERIDIN-1-YLOXY POUR UTILISATIONS DANS DES UNITÉS DE STOCKAGE DE CHARGE

(30) Priorität: 29.11.2019 EP 19212540
(43) Veröffentlichungstag der Anmeldung: 05.10.2022
(73) Patentinhaber: Evonik Operations GmbH, 45128 Essen (DE)
(72) Erfinder: GÄRTNER, Felix, 45721 Haltern am See (DE); NISSEN, Felix, 48301 Nottuln (DE)
(74) Vertreter: Evonik Patent Association
(86) Internationale Anmeldenummer: PCT/EP2020/064503
(87) Internationale Veröffentlichungsnummer: WO 2021/104686

(56) Entgegenhaltungen:
- WO-A1-2018/028830
- JP-A- H 083 136
- RU-C1- 2 114 830
- US-A1- 2018 072 669
- TOBIAS JANOSCHKA ET AL: "An Aqueous Redox-Flow Battery with High Capacity and Power: The TEMPTMA/MV System", ANGEWANDTE CHEMIE, INTERNATIONAL EDITION, Bd. 55, Nr. 46, 18. Oktober 2016 (2016-10-18), Seiten 14427-14430, XP055494176, DE ISSN: 1433-7851, DOI: 10.1002/anie.201606472 in der Anmeldung erwähnt & Tobias Janoschka ET AL: "Supporting Information Aqueous Redox-Flow Battery with High Capacity and Power: The TEMPTMA/MV System", , 18. Oktober 2016 (2016-10-18), Seiten 1-14, XP055667611, DOI: 10.1002/anie.201606472 Gefunden im Internet: URL:https://onlinelibrary.wiley.com/doi/fu ll/10.1002/anie.201606472 [gefunden am 2020-02-12]

## Beschreibung

Die vorliegende Erfindung betrifft ein Verfahren zur Herstellung wässriger Lösungen von 4-Ammonium-alkylpiperidin-1-yloxylsalzen. Darin erfolgt eine Umsetzung des Eduktes, eines 4-Alkylammoniumsalzes des 2,2,6,6-Tetramethylpiperidins, mit H₂O₂ in wässriger Lösung enthaltend CO₂. Das erfindungsgemäße Verfahren zeichnet sich durch den sparsamen Einsatz der Edukte aus. Die durch dieses Verfahren erhaltenen Lösungen eignen sich außerdem besonders gut zur Verwendung in Ladungsspeichereinheiten, da der Gehalt an störenden Fremdionen in den Lösungen durch das erfindungsgemäße Vorgehen minimiert werden kann.

### Hintergrund der Erfindung

Die Synthese von 4-Ammonium-alkylpiperidin-1-yloxysalzen ist von großer Bedeutung für das Gebiet der organischen Batterien, in welchen solche Verbindungen als Elektrolytmaterial eingesetzt werden. Die Synthese solcher Materialien ist vielfach im Stand der Technik beschrieben. Der übliche Syntheseweg verläuft dabei über zwei Stufen:
Stufe **1:** 4-Aminoalkylpiperidin wird mit Alkylhalogenid oder einem anderen Alkylierungsmittel zum 4-Ammonium-alkylpiperidinylsalz umgesetzt.
Stufe **2:** Das 4-Ammonium-alkylpiperidinylsalz wird durch Oxidation mit einer Perverbindung wie beispielsweise H₂O₂ zum 4-Ammonium-alkylpiperidin-1-yloxysalz umgesetzt.

In der Stufe **1** wird üblicherweise das entsprechende Alkyljodid eingesetzt, wie etwa von C. Francavilla et al., Bioorganic and Medicinal Chemistry Letters 2011, 21, 3029-3033 auf Seite 3031 in Schema 6 bei der Umsetzung von Verbindung **38** in Verbindung **39** beschrieben. Die gleiche Reaktion wird auch in Janoschka et al., Angew. Chem. Int. Ed. 2016, 55, 14427-14430 (im Folgenden "Janoschka et *al."*) auf Seite 14429 in Schema 1 bei der Umsetzung von Verbindung **2** in Verbindung **3** beschrieben. US 2018/0072669 A1 beschreibt auf Seite 35 die entsprechende Umsetzung mit Methyljodid.

B. Bales et al., J. Phys. Chem. A 2009, 113, 9295-9303 beschreiben auf Seite 9298, Schema 1, die Umsetzung eines deuterierten 4-Aminoalkylpiperidinderivats mit n-Dodecylbromid zum entsprechenden Bromidsalz.

B. K. Sinha & C. F. Chignell, J. Med. Chem. 1975, 18, 669-673 (im Folgenden "Sinha & Chignell") beschreiben die entsprechende Umsetzung von 4-Dimethylamino-2,2,6,6-tetramethylpiperidin mit 1,6-Dibromhexan bzw. 1,10-Dibromdecan zum jeweiligen Bromidsalz.

Sowohl Janoschka et *al.* als auch Sinha & Chignell und US 2018/0072669 A1 (Absatz [0314]) beschreiben außerdem die weitere Stufe (Stufe **2**), also die Oxidation der sekundären Aminogruppe zum jeweiligen N-O·-Radikal. Als Oxidationsmittel werden H₂O₂ und Natriumwolframat eingesetzt.

JPH08-3136 und RU 2114830 C1 behandeln die Überführung nichtionischer 4-Oxo- oder 4-OH-Derivate des 2,2,6,6-Tetramethylpiperidins in das entsprechende Nitroxyl-Radikal mittels Umsetzung mit H₂O₂ in Gegenwart von CO₂.

Die im Stand der Technik beschriebenen Verfahren, welche der oben genannten Stufe 1 entsprechen, wenden üblicherweise Alkylbromid oder Alkyljodid als Alkylierungsmittel an.

Dies ist insofern von Nachteil, als in den Fällen, in denen ein Chloridsalz gewünscht ist, ein Anionenaustausch des Jodids oder Bromids durch Chlorid erfolgen muss, was einen zusätzlichen Schritt erfordert und die gesamte Synthese somit aufwendiger macht.

Dieser Nachteil kann durch die direkte Umsetzung des **4**-Aminopiperidins mit Alkylchloriden und Sulfaten, wie in der WO 2018/028830 A1 und von Janoschka et al. beschrieben, umgangen werden.

In diesen Offenbarungen erfolgt im Einzelnen eine direkte Umsetzung mit Methylchlorid. Der Einsatz von Methylchlorid hat allerdings wiederum den Nachteil, dass Methylchlorid weniger reaktiv ist als das entsprechende Methyljodid oder Methylbromid und die Reaktion somit langsamer verläuft. Ein weiterer Nachteil der von WO 2018/028830 A1 und von Janoschka et al. beschriebenen Synthese ist außerdem, dass die Stufe **2,** also die Oxidation zum entsprechenden N-O·-Radikal, unter Verwendung von Erdalkalisulfaten mit einem großen Überschuss an H₂O₂ erfolgt.

Diese Reaktionsführung ist schon allein aufgrund des hohen Ressourcenverbrauchs nachteilig. Daneben wurde beobachtet, dass die im Stand der Technik beschriebene Oxidation zu Niederschlägen führt, welche die Aufarbeitung *per se* verkomplizieren. Durch die Verwendung verschiedener Reaktanden wird in die Produktlösung außerdem eine Vielzahl von ionischen Verbindungen eingetragen, die die Löslichkeit von **4**-Ammonium-alkylpiperidin-1-yloxysalzen in wässriger Lösung vermindern (siehe Janoschka et al., Supporting Information*,* Abbildung **S1**). Um eine maximale Löslichkeit zu erreichen, umfassen Elektrolytlösungen idealerweise ausschließlich die als Ladungsträger aktiven Verbindungen wie zum Beispiel **4**-Ammonium-alkylpiperidin-1-yloxysalzen, während die Anwesenheit weiterer Ionen wie Erdalkalimetallionen unerwünscht ist.

Die erfindungsgemäße Aufgabe besteht demnach darin, ein Verfahren Herstellung von 4-Ammonium-alkylpiperidin-1-yloxylsalzen zur Verfügung zu stellen, welches die beschriebenen Nachteile der herkömmlichen Verfahren nicht aufweist und insbesondere die unerwünschte Salzfracht in der Elektrolytlösung minimiert.

Es wurde nun überraschend ein Verfahren gefunden, welches die erfindungsgemäße Aufgabe löst.

### Detaillierte Beschreibung der Erfindung

Die vorliegende Erfindung betrifft demnach ein Verfahren zur Herstellung einer wässrigen Elektrolytlösung **L₁** umfassend mindestens eine Verbindung der Formel **(I)** oder **(II)**, bevorzugt mindestens eine Verbindung der Formel **(I)**, mit 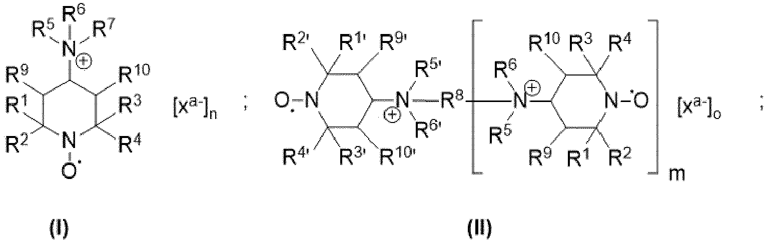
wobei R¹, R², R³, R⁴, R¹', R²', R³', R⁴' unabhängig voneinander jeweils ein C₁-C₆-Alkylrest, insbesondere ein C₁-C₄-Alkylrest, bevorzugt ein C₁-C₂-Alkylrest, und bevorzugter alle jeweils Methyl sind,
wobei R⁹, R¹⁰, R⁹', R¹⁰' unabhängig voneinander jeweils aus der Gruppe bestehend aus Wasserstoff, C₁-C₆-Alkylrest, insbesondere aus der Gruppe bestehend aus Wasserstoff, C₁-C₄-Alkylrest, bevorzugt aus der Gruppe bestehend aus Wasserstoff, Methyl, Ethyl ausgewählt sind, und bevorzugter alle Wasserstoff sind,
wobei R⁵, R⁶, R⁵', R⁶' unabhängig voneinander jeweils aus der Gruppe bestehend aus Wasserstoff, Alkyl, Cycloalkyl, Aryl, Aralkyl, Heterocyclyl ausgewählt sind, wobei die Reste Alkyl, Cycloalkyl, Aryl, Aralkyl, Heterocyclyl mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid, Halogen substituiert sein können, wobei außerdem Alkyl durch ein oder mehrere, nicht direkt zueinander benachbarte zweiwertige Reste ausgewählt aus der Gruppe bestehend aus Sauerstoff, Imino unterbrochen sein kann,
wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei außerdem R⁵' und R⁶' zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei insbesondere R⁵, R⁶, R⁵', R⁶' unabhängig voneinander jeweils aus der Gruppe bestehend aus Wasserstoff, Alkyl, wobei Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, wobei außerdem R⁵ und R⁶ eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können, und wobei außerdem R⁵' und R⁶' zusammen eine Alkylengruppe die durch ein oder mehrere, nicht direkt zueinander benachbarte zweiwertige Reste ausgewählt aus der Gruppe bestehend aus Sauerstoff, Imino unterbrochen sein kann, bilden können,
wobei bevorzugt R⁵, R⁶, R⁵', R⁶' unabhängig voneinander jeweils aus der Gruppe bestehend aus C₁-C₆-Alkyl, wobei C₁-C₆-Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind, wobei außerdem R⁵ und R⁶ eine C₁-C₁₀-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können, und wobei außerdem R⁵' und R⁶' zusammen eine C₁-C₁₀-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei noch bevorzugter R⁵, R⁶, R⁵', R⁶' unabhängig voneinander jeweils eine C₁-C₅-Alkylgruppe, die durch ein oder mehrere, nicht direkt zueinander benachbarte zweiwertige Reste ausgewählt aus der Gruppe bestehend aus Sauerstoff, Imino unterbrochen sein kann, sind, wobei außerdem R⁵ und R⁶ eine C₁-C₆-Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können, wobei außerdem R⁵' und R⁶' zusammen eine C₁-C₆-Alkylengruppe die durch ein oder mehrere, nicht direkt zueinander benachbarte zweiwertige Reste ausgewählt aus der Gruppe bestehend aus Sauerstoff, Imino unterbrochen sein kann, bilden können,
wobei noch mehr bevorzugter R⁵, R⁶, R⁵', R⁶' unabhängig voneinander jeweils aus der Gruppe bestehend aus Methyl, Ethyl, *n*-Propyl, *n*-Butyl, -CH₂CH₂(NH)CH₃, -CH₂CH₂(NCH₃)CH₂CH₂(NH)CH₃ ausgewählt sind, wobei außerdem R⁵ und R⁶ zusammen eine Gruppe ausgewählt aus Piperidin, Morpholin, Piperazin, Pyrrolidin bilden können und wobei außerdem R⁵' und R⁶' zusammen eine Gruppe ausgewählt aus Piperidin, Morpholin, Piperazin, Pyrrolidin bilden können,
wobei noch viel mehr bevorzugter R⁵, R⁶, R⁵', R⁶' unabhängig voneinander jeweils aus der Gruppe bestehend aus Methyl, Ethyl, *n*-Propyl ausgewählt sind und am allerbevorzugtesten R⁵, R⁶, R⁵', R⁶' jeweils Methyl sind,
wobei R⁷ ein Alkylrest ist, der mit ein oder mehreren Resten ausgewählt aus der Gruppe bestehend aus Alkoxy, Hydroxy, Nitro, Carboxyl, Carboxylester, Carboxylamid, Sulfonat, Sulfonsäureester, Sulfonamid, Halogen substituiert sein kann und der durch ein oder mehrere, nicht direkt zueinander benachbarte zweiwertige Reste ausgewählt aus der Gruppe bestehend aus Sauerstoff, Imino unterbrochen sein kann,
wobei R⁷ insbesondere C₁-C₆-Alkyl, welches durch ein oder mehrere, nicht direkt zueinander benachbarte zweiwertige Reste ausgewählt aus der Gruppe bestehend aus Sauerstoff, Imino unterbrochen sein kann, ist,
wobei R⁷ bevorzugt C₁-C₅-Alkyl, welches durch ein oder mehrere, nicht direkt zueinander benachbarte zweiwertige Reste ausgewählt aus der Gruppe bestehend aus Sauerstoff, Imino unterbrochen sein kann, ist,
wobei R⁷ bevorzugter aus der Gruppe bestehend aus Methyl, Ethyl, *n*-Propyl, *n*-Butyl, -CH₂CH₂(NH)CH₃, -CH₂CH₂(NCH₃)CH₂CH₂(NH)CH₃ ausgewählt ist,
wobei R⁷ noch bevorzugter aus der Gruppe bestehend aus Methyl, Ethyl, *n*-Propyl ausgewählt ist,
wobei R⁷ noch mehr bevorzugter aus der Gruppe bestehend aus Methyl, Ethyl ausgewählt ist,
und R⁷ am bevorzugtesten Methyl ist,
wobei R⁸ eine p-wertige organische Brückengruppe ist, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann, und die durch ein oder mehrere, nicht direkt zueinander benachbarte zweiwertige Reste ausgewählt aus der Gruppe bestehend aus Sauerstoff, Imino unterbrochen sein kann,
wobei R⁸ insbesondere eine Alkylengruppe, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann und die durch ein oder mehrere, nicht direkt zueinander benachbarte zweiwertige Reste ausgewählt aus der Gruppe bestehend aus Sauerstoff, Imino unterbrochen sein kann, ist,
wobei R⁸ bevorzugt eine Alkylengruppe, die durch ein oder mehrere, nicht direkt zueinander benachbarte zweiwertige Reste ausgewählt aus der Gruppe bestehend aus Sauerstoff, Imino unterbrochen sein kann, ist,
wobei R⁸ bevorzugter eine Alkylengruppe ist, noch bevorzugter eine C₁-C₁₀-Alkylengruppe, noch mehr bevorzugter eine C₁-C₆-Alkylengruppe, noch viel mehr bevorzugter eine C₂-C₆-Alkylengruppe, am bevorzugtesten aus der Gruppe bestehend aus Ethylen (also "-CH₂CH₂-), Butylen (also "-CH₂CH₂CH₂CH₂-"), Hexamethylen (also "-CH₂CH₂CH₂CH₂CH₂CH₂-") ausgewählt ist, und am allerbevorzugtesten Hexamethylen ist,
a die Wertigkeit des Anions X^{a-} angibt und eine ganze Zahl von 1 bis 10.000, insbesondere 1 bis 50, bei Verbindungen der Formel **(I)** und **(III)** bevorzugt 1 bis 3, bevorzugter 1 bis 2 und noch bevorzugter 1 bedeutet und bei Verbindungen der Formel **(II)** und **(IV)** bevorzugt 1 bis 6, noch bevorzugter 1 bis 5, noch mehr bevorzugter 1 bis 4, noch mehr bevorzugter 1 bis 3 und am bevorzugtesten 1 bis 2 bedeutet, am allerbevorzugtesten 1 bedeutet,
p eine ganze Zahl von 2 bis 6, insbesondere 2 bis 5, noch bevorzugter 2 bis 4, noch mehr bevorzugter 2 bis 3 und am bevorzugtesten 2 bedeutet,
m eine ganze Zahl mit dem Wert "p - 1" bedeutet,
n eine Zahl mit dem Wert "1 / a" bedeutet,
o eine Zahl mit dem Wert "(1 + m) / a" bedeutet, und
X^{a-} ein organisches Anion, ein anorganisches Anion oder eine Mischung dieser Anionen ist, insbesondere ein anorganisches Anion ist, wobei X^{a-} bevorzugt aus der Gruppe bestehend aus Halogenidionen (Wertigkeit a = 1), Hydroxyionen (Wertigkeit a = 1), Phosphationen (Wertigkeit a = 3), Sulfationen (Wertigkeit a = 2), Perchlorationen (Wertigkeit a = 1), Hexafluorophosphationen (Wertigkeit a = 1), Tetrafluoroborationen (Wertigkeit a = 1) ausgewählt ist, bevorzugter aus der Gruppe bestehend aus Halogenidionen, Sulfatanion ausgewählt ist, noch bevorzugter aus der Gruppe bestehend aus Chlorid, Methylsulfat ausgewählt ist,
gekennzeichnet dadurch, dass
   (a) mindestens eine Verbindung der Formel **(III)** oder **(IV)** mit 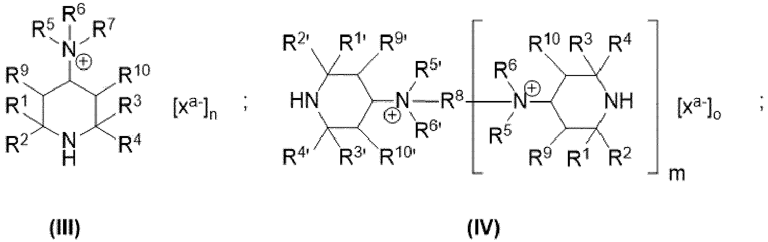 mit H₂O₂ in einer COz-haltigen, wässrigen Lösung **L₂** zu einer Verbindung der Formel **(I)** bzw. **(II)** umgesetzt wird und
   (b) nach der Umsetzung gemäß Schritt (a) H₂O₂, falls dieses dann noch in der wässrigen Lösung **L₂** enthalten ist, aus **L₂** entfernt wird, und gegebenenfalls auch CO₂ teilweise aus **L₂** entfernt wird,
      wodurch eine CO₂-haltige, wässrige Lösung **L₃** umfassend mindestens eine Verbindung der Formel **(I)** bzw. **(II)** erhalten wird,
   (c) ein saurer pH-Wert in **L₃** eingestellt wird, wodurch CO₂ mindestens teilweise aus **L₃** entfernt und die wässrige Elektrolytlösung **L₁** erhalten wird.

In der bevorzugten Ausführungsform, in der im erfindungsgemäßen Verfahren eine wässrigen Lösung **L₁** mindestens einer Verbindung der Formel **(I)** hergestellt wird und deshalb eine Verbindung der Formel **(III)** eingesetzt wird, gelten die vorgenannten Definitionen der Reste R¹, R², R³, R⁴, R⁵, R⁶, R⁷, R⁹, R¹⁰, X^{a-} und der Variablen n entsprechend.

Das erfindungsgemäße Verfahren hat den überraschenden Vorteil, dass keine aufwendige Aufarbeitung wie Filtration der wässrigen Lösung **L₁** durchgeführt werden muss, um zu einem Produkt zu gelangen, welches in Ladungsspeichereinheiten angewendet werden kann. Dies war überraschend im Lichte des Standes der Technik wie JP-H08-3136 und RU 2114830 C1. Die Erfindung beruht darauf, dass durch den Einsatz von CO₂ als Katalysator keine Salze eingetragen werden, wodurch die erhaltenen Lösungen eine geringere Salzfracht haben, insbesondere dann, wenn entsprechende Methoden zur Entfernung des H₂O₂ angewandt werden.

Das erfindungsgemäße Verfahren wird bevorzugt kontinuierlich durchgeführt.

### 1. Schritt (a)

In Schritt (a) des erfindungsgemäßen Verfahrens wird mindestens eine Verbindung der Formel **(III)** oder **(IV)** mit H₂O₂ in einer CO₂-haltigen, wässrigen Lösung **L₂** zu einer Verbindung der Formel **(I)** bzw. **(II)** umgesetzt. Demnach wird eine wässrige Lösung **L₂** eingesetzt.

Die wässrige Lösung **L₂** umfasst mindestens eine Verbindung der Formel **(III)** oder **(IV),** bzw. in den Ausführungsformen des erfindungsgemäßen Verfahrens, in denen eine wässrige Lösung **L₁** umfassend mindestens eine Verbindung der Formel **(I)** hergestellt wird, mindestens eine Verbindung der Formel **(III)**.

"Zu einer Verbindung der Formel **(I)** bzw. **(II)** umgesetzt wird" ist so zu verstehen, dass, wenn eine Verbindung der Formel **(III)** in Schritt (a) des erfindungsgemäßen Verfahrens eingesetzt wird, dann eine Verbindung der Formel **(I)** als Produkt erhalten wird, und wenn eine Verbindung der Formel **(IV)** in Schritt (a) des erfindungsgemäßen Verfahrens eingesetzt wird, dann eine Verbindung der Formel **(II)** als Produkt erhalten wird.

Insbesondere wird Schritt (a) des erfindungsgemäßen Verfahrens dabei bei einem pH-Wert von 7.5 bis < 10.5, bevorzugt 8 bis < 10.5, bevorzugter 8.5 bis < 10.5, noch bevorzugter 9 bis 10, noch mehr bevorzugter 9.5 bis 10 durchgeführt.

Insbesondere wird Schritt (a) des erfindungsgemäßen Verfahrens dabei bei einer Temperatur von 20 °C bis 100 °C, bevorzugter 30 °C bis 80 °C, noch bevorzugter 40 °C bis 75 °C, am bevorzugtesten 60 °C bis 75 °C durchgeführt.

Insbesondere wird Schritt (a) des erfindungsgemäßen Verfahrens dabei bei einem Druck von 0.5 bar bis 2 bar, bevorzugt bei 1 bar durchgeführt.

Die wässrige Lösung **L₂** enthält CO₂. CO₂ fungiert als Katalysator in Schritt (a) des vorliegenden Verfahrens. Die CO₂-haltige, wässrige Lösung **L₂** kann nach dem Fachmann bekannten Verfahren bereitgestellt werden. Bevorzugt wird vor Schritt (a) CO₂ in Form von Trockeneis und/oder gasförmig in Wasser gelöst, wodurch eine CO₂-haltige wässrigen Lösung **L₂** erhalten wird, die dann in Schritt (a) des erfindungsgemäßen Verfahrens eingesetzt wird.

Alternativ kann auch zuerst die mindestens eine Verbindung der Formel **(I)** bzw. **(II)** in Wasser gelöst werden und dann CO₂ in Form von Trockeneis und/oder gasförmig darin gelöst werden, was insbesondere dann unmittelbar den Schritt (a) des erfindungsgemäßen Verfahrens initiiert.

Es versteht sich von selbst, dass das Merkmal "CO₂-haltig" bedeutet, dass CO₂ in verschiedener chemischer Form in der wässrigen Lösung **L₂** enthalten sein kann. Dies umfasst insbesondere neben in der wässrigen Lösung **L₂** hydratisierten CO₂-Molekülen (Kohlensäure) auch CO₂ in Form von Hydrogencarbonatanionen und Carbonatanionen. Im Folgenden wird unter "in der wässrigen Lösung **L₂** gelöstem CO₂" die Gesamtmenge der in der wässrigen Lösung **L₂** enthaltenen hydratisierten CO₂-Moleküle, Hydrogencarbonatanionen, Carbonatanionen verstanden.

In einer bevorzugten Ausführungsform des erfindungsgemäßen Verfahrens liegt das molare Verhältnis der Stoffmenge des in der wässrigen Lösung **L₂** gelösten CO₂ zur gesamten Stoffmenge aller in Schritt (a) des erfindungsgemäßen Verfahrens eingesetzten Verbindungen der Formeln **(III)** und **(IV)** im Bereich 10 : 1 bis 1 : 1000, bevorzugter im Bereich 1 : 1 zu 1 : 100, noch bevorzugter im Bereich 1 : 10 bis 1 : 50, am bevorzugtesten bei 1 : 21.

In einer alternativen bevorzugten Ausführungsform ergibt sich die Gesamtmenge des in der wässrigen Lösung **L₂** gelösten CO₂ bezogen auf die Menge der in Schritt (a) des erfindungsgemäßen Verfahrens eingesetzten Verbindungen der Formel **(III)** bzw. **(IV)** wie folgt:
In dieser alternativen bevorzugten Ausführungsform enthält die wässrige Lösung **L₂** pro Mol an in Schritt (a) des erfindungsgemäßen Verfahrens eingesetzter Verbindung der Formel **(III)** 0.001 bis 10 Mol, bevorzugt 0.01 bis 1 Mol, bevorzugter 0.025 bis 0.1 Mol, am bevorzugtesten 1/21 Mol an gelöstem CO₂, und zusätzlich pro Mol an in Schritt (a) des erfindungsgemäßen Verfahrens eingesetzter Verbindung der Formel (IV) p x [0.001 bis 10] Mol, bevorzugt p x [0.01 bis 1] Mol, bevorzugter p × [0.025 bis 0.1] Mol, am bevorzugtesten p × 1/21 Mol an gelöstem CO₂.

Es ist selbstverständlich dass, wenn im erfindungsgemäßen Verfahren mehrere Verbindungen der Formel **(IV),** welche sich durch den Wert von m bzw. p unterscheiden, eingesetzt werden, dass dann die bevorzugte Menge für jede Gruppe mit dem selben Wert für p wiederholt werden und dann die pro Gruppe berechneten Mengen an gelöstem CO₂ addiert werden müssen.

Bei Verfahren zur Herstellung von Verbindungen der Formel **(I)** enthält die wässrige Lösung **L₂** pro Mol an in Schritt (a) des erfindungsgemäßen Verfahrens eingesetzter Verbindung der Formel **(III)** insbesondere 0.001 bis 10 Mol, bevorzugt 0.01 bis 1 Mol, bevorzugter 0.025 bis 0.1 Mol, am bevorzugtesten 1/21 Mol an gelöstem CO₂.

Im erfindungsgemäßen Verfahren wird in Schritt (a) bevorzugt eine Verbindung der Formel **(III)** eingesetzt, so dass das erfindungsgemäße Verfahren eines zur Herstellung einer Verbindung der Formel **(I)** ist. Dabei sind noch bevorzugter R¹, R², R³, R⁴, R⁵, R⁶, R⁷ jeweils Methyl und R⁹, R¹⁰ jeweils Wasserstoff. Bei X^{a-} handelt es sich dann insbesondere um ein einwertiges organisches Anion, bevorzugt Methylsulfat oder ein Halogenid, welches noch bevorzugter aus der Gruppe bestehend aus Chlorid, Bromid, Jodid ausgewählt ist. Am bevorzugtesten ist X^{a-} dann Chlorid. Die Variablen a und n haben dann jeweils den Wert 1.

Im erfindungsgemäßen Verfahren wird H₂O₂ eingesetzt, insbesondere als wässrige Lösung mit einem Gehalt von 20 bis 50 Gew.-%, bevorzugt 30 bis 50 Gew.-% H₂O₂ in Wasser.

Im erfindungsgemäßen Verfahren liegt insbesondere das molare Verhältnis des Gesamtgewichts aller in Schritt (a) eingesetzten Verbindungen der Formeln **(III)** und **(IV)** zum Gesamtgewicht des in Schritt (a) eingesetzten H₂O₂ im Bereich 9 : 1 bis 1 : 9, bevorzugt 1 : 1.05 bis 1 : 9, bevorzugter 1 : 1.2 bis 1 : 5, noch bevorzugter 1 : 1.3 bis 1 : 3, noch bevorzugter 1 : 1.4 bis 1 : 2, am bevorzugtesten 1 : 1 bis 1 : 1.5.

In einer alternativen bevorzugten Ausführungsform wird die Menge des in Schritt (a) eingesetzten H₂O₂ bezogen auf die Menge der in Schritt (a) des erfindungsgemäßen Verfahrens eingesetzten Verbindungen der Formel **(III)** bzw. **(IV)** bestimmt.

In dieser alternativen bevorzugten Ausführungsform werden pro Mol an in Schritt (a) eingesetzter Verbindung der Formel **(III)** 1.1 bis 9 Mol H₂O₂, bevorzugt 1.25 bis 4 Mol H₂O₂, bevorzugter 1.4 bis 2.67 Mol H₂O₂, noch bevorzugter 1.5 bis 2.5 Mol H₂O₂, am bevorzugtesten 1.50 Mol H₂O₂ in Schritt (a) eingesetzt und pro Mol an in Schritt (a) eingesetzter Verbindung der Formel **(IV)** p × [1.1 bis 9] Mol H₂O₂, bevorzugt p × [1.25 bis 4] Mol H₂O₂, bevorzugter p × [1.4 bis 2.67] Mol H₂O₂, noch bevorzugter p × [1.5 bis 2.5] Mol H₂O₂, am bevorzugtesten p × [1.50] Mol H₂O₂ in Schritt (a) eingesetzt.

Es ist selbstverständlich dass, wenn im erfindungsgemäßen Verfahren mehrere Verbindungen der Formel **(IV),** welche sich durch den Wert von m bzw. p unterscheiden, eingesetzt werden, dass dann die bevorzugte Menge an H₂O₂ für jede Gruppe mit dem selben Wert für p wiederholt werden und dann die pro Gruppe berechneten Mengen an H₂O₂ addiert werden müssen.

Bei Verfahren zur Herstellung von Verbindungen der Formel **(I)** werden insbesondere pro Mol an in Schritt (a) eingesetzter Verbindung der Formel **(III)** 1.1 bis 9 Mol H₂O₂, bevorzugt 1.25 bis 4 Mol H₂O₂, bevorzugter 1.4 bis 2.67 Mol H₂O₂, noch bevorzugter 1.5 bis 2.5 Mol H₂O₂, am bevorzugtesten 1.50 Mol H₂O₂ in Schritt (a) eingesetzt.

Das erfindungsgemäße Verfahren wird in der wässrigen Lösung **L₂** durchgeführt. Dies ist beim Einsatz von Wasserstoffperoxid als Peroxid schon allein dadurch der Fall, dass dabei stets Wasser mit in die Reaktion eingetragen werden. Der Anteil von Wasser in der wässrigen Lösung **L₂** liegt dabei insbesondere im Bereich 10 Gew.-% bis 90 Gew.-%, bevorzugt 20 Gew.-% bis 80 Gew.-%, bevorzugter 30 Gew.-% bis 70 Gew.-%, jeweils bezogen auf das Gesamtgewicht der wässrigen Lösung **L₂**.

In einer bevorzugten Ausführungsform des erfindungsgemäßen Verfahrens umfasst die wässrige Lösung **L₂**, gerade wenn das erfindungsgemäße Verfahren in Edelstahlreaktoren durchgeführt wird, Stabilisatoren für H₂O₂. Solche Stabilisatoren sind dem Fachmann bekannt und beispielsweise beschrieben in US 4,294,575, US 4,304,762, CA 1,152,292, US 4,034,064, US 4,061,721, US 4,362,706. Insbesondere werden Stabilisatoren ausgewählt aus der Gruppe bestehend aus Pyrophsophaten, bevorzugt Natriumpyrophosphaten, Phosphaten, Stannaten, Magnesiumionen, Silicationen, aminsubstituierten Organophosphonsäuren und deren Alkalisalzen.

Es versteht sich von selbst, dass der Schritt (a) des erfindungsgemäßen Verfahrens möglichst vollständig ablaufen muss, bevor der Folgeschritt (b) bzw. (c) erfolgt.

Es ist deshalb bevorzugt, dass sich in Schritt (a) des erfindungsgemäßen Verfahrens mindestens 10 Mol.-%, bevorzugt mindestens 60 Mol.-%, bevorzugter mindestens 70 Mol.-%, noch bevorzugter mindestens 80 Mol.-%, noch mehr bevorzugter mindestens 90 Mol.-%, am bevorzugtesten mindestens 99 Mol.-% aller darin eingesetzten Verbindungen der Formeln **(III)** und **(IV)** zu den Verbindungen der Formel **(I)** und **(II)** umsetzen. Erst wenn diese Umsetzung erreicht ist, erfolgen in dieser bevorzugten Ausführungsform die Schritte (b) bzw. (c). Am bevorzugtesten ist die Umsetzung der in Schritt (a) des erfindungsgemäßen Verfahrens eingesetzten Verbindungen der Formeln **(III)** und **(IV)** zu den Verbindungen der Formel **(I)** und **(II)** vollständig, bevor zu Schritten (b) bzw. (c) übergegangen wird.

Nach Beendigung von Schritt (a) des erfindungsgemäßen Verfahrens umfasst die dann erhaltene Lösung gegebenenfalls H₂O₂, insbesondere wenn dieses in Schritt (a) im molaren Überschuss zur molaren Gesamtmenge der Verbindungen **(III)** und **(IV)** eingesetzt wurde.

Die vorliegende Erfindung zeichnet sich nun dadurch aus, dass im Vergleich zum im Stand der Technik wie WO 2018/028830 A1 beschriebenen Verfahren eine Elektrolytlösung erhalten wird, die eine geringere Salzfracht und damit eine höhere Löslichkeit der Verbindung **(I)** bzw. **(II)** aufweist. So Der Grund dafür liegt im Einsatz der CO₂-haltigen Lösung, welche eine rückstandsfreie Entfernung aus der Lösung ermöglicht.

### 2. Schritt (b)

Schritt (b) erfolgt nach der Umsetzung gemäß Schritt (a). in Schritt (b) wird H₂O₂, falls dieses dann noch in der wässrigen Lösung **L₂** enthalten ist, aus **L₂** entfernt wird, und gegebenenfalls auch CO₂ teilweise aus **L₂** entfernt.

Schritt (b) des erfindungsgemäßen Verfahrens wird demnach dann durchgeführt, falls die wässrige Lösung **L₂** nach der Umsetzung der mindestens einen Verbindung der Formel **(III)** oder **(IV)** mit H₂O₂ zur mindestens einen Verbindung der Formel **(I)** oder **(II)** noch H₂O₂ enthält.

Das ist insbesondere dann der Fall, wenn in Schritt (a) des erfindungsgemäßen Verfahrens H₂O₂ im molaren Überschuss bezogen auf die Gesamtmenge an Verbindungen **(III)** und **(IV)** eingesetzt wird.

In diesem Fall wird H₂O₂ aus der wässrigen Lösung **L₂** in Schritt (b) entfernt. "Entfernung" bedeutet in diesem Fall insbesondere, dass die nach Schritt (b) erhaltene wässrige Lösung **L₃** einen Gehalt an H₂O₂ aufweist, der < 10 Mol.-%, bevorzugt < 5 Mol.-%, bevorzugter < 1 Mol.-%, noch bevorzugter < 0.1 Mol.-% beträgt, bezogen auf den Gehalt an H₂O₂ in der wässrigen Lösung **L₂** nach Durchführung des Schrittes (a) des erfindungsgemäßen Verfahrens.

Die Entfernung von H₂O₂ im Schritt (b) des erfindungsgemäßen Verfahrens erfolgt insbesondere über zwei bevorzugte alternative Verfahren:
2.1 In einer ersten bevorzugten Ausführungsform des Schritts (b) wird nach der Umsetzung gemäß Schritt (a) H₂O₂ durch Einstellen der wässrigen Lösung **L₂** auf einen pH-Wert von ≥ 10.5 entfernt. Noch bevorzugter wird ein pH-Wert im Bereich von 10.5 bis 12, noch mehr bevorzugter 10.6 bis 11.5, am bevorzugtesten ein pH von 11 eingestellt.

Die Einstellung des pH-Wertes der wässrigen Lösung **L₂** in Schritt (b) erfolgt dabei insbesondere durch Zugabe einer Base, insbesondere ausgewählt aus der Gruppe bestehend aus Alkalimetallhydroxid, Erdalkalimetallhydroxid.

Am bevorzugtesten wird dabei NaOH als Base eingesetzt.

2.2 In einer zweiten bevorzugten Ausführungsform des Schritts (b) wird nach der Umsetzung gemäß Schritt (a) H₂O₂ durch Umsetzen der wässrigen Lösung **L₂** in Gegenwart eines Katalysators entfernt. Der Katalysator ist dabei bevorzugt aus der Gruppe bestehend aus Trägermetallkatalysatoren, Metallvollkatalysatoren, Metalloxide, Enzyme ausgewählt.

2.2.1 Trägermetallkatalysatoren sind dem Fachmann bekannt und insbesondere in der EP 0 302 020 A2 beschrieben.

Trägermetallkatalysatoren umfassen mindestens ein, bevorzugt elementar vorliegendes, Metall M_{T}.

Das Metall M_{T} ist dabei insbesondere ausgewählt aus der Gruppe bestehend aus V, Cr, Mo, Mn, Ni, Pd, Pt, Fe, Ru, Os, Co, Rh, Ir, Cu; bevorzugt ausgewählt aus der Gruppe bestehend aus V, Cr, Mo, Mn, Ni, Pd, Pt, Fe, Ru, Os, Co, Rh, Ir, Cu; bevorzugter ausgewählt aus der Gruppe bestehend aus Cr, Mo, Mn, Ni, Pd, Pt, Ru, Rh; noch bevorzugter ausgewählt aus der Gruppe bestehend aus Cr, Ni, Pd, Pt; am bevorzugtesten ausgewählt aus der Gruppe bestehend aus Pt, Pd.

Bei einem Trägermetallkatalysator ist das Metall M_{T} auf einem dem Fachmann bekannten Träger aufgebracht, welcher insbesondere ausgewählt aus der Gruppe bestehend aus Aktivkohle, Calciumcarbonat, Aluminiumoxid, Titandioxid, bevorzugt aus der Gruppe bestehend aus Aluminiumoxid, Aktivkohle ausgewählt ist. Am bevorzugtesten ist als Träger Aluminiumoxid, Al₂O₃.

Der Anteil an Metall **M_{T}** im Trägermetallkatalysator ist dabei nicht besonders beschränkt und liegt insbesondere im Bereich 0.1 bis 30 Gew.-%, bevorzugt 1 bis 10 Gew.-%, bevorzugter 5 Gew.-%. Dabei bedeutet Gew.-% das Gesamtgewicht aller vom jeweiligen Trägermetallkatalysator umfassten Metalle **M_{T}** bezogen auf das Gesamtgewicht des vom jeweiligen Trägermetallkatalysator umfassten Trägers.

Wird in Schritt (b) des erfindungsgemäßen Verfahrens H₂O₂ aus **L₂** mit einem Trägermetallkatalysator entfernt, wird der Trägermetallkatalysator zur wässrigen Lösung **L₂** zugegeben und H₂O₂ an diesem Trägerkatalysator zu H₂O und O₂ disproportioniert.

Dabei wird die Temperatur in Schritt (b) bevorzugt bei 20 °C bis 80 °C, bevorzugter bei 40 °C bis 70 °C, noch bevorzugter bei 50 °C bis 60 °C, am bevorzugtesten bei 60 °C eingestellt.

Insbesondere beträgt das Gewicht (Massenanteil) des zugegebenen Trägerkatalysators 0.1 % bis 5 %, bevorzugter 0.5 % bis 2 %, bezogen auf das Gesamtgewicht der wässrigen Lösung **L₂**. Bevorzugt, insbesondere bei kontinuierlicher Fahrweise des erfindungsgemäßen Verfahrens, wird bei Umsetzen der wässrigen Lösung **L₂** in Gegenwart eines Trägermetallkatalysators in Schritt (b) die wässrige Lösung **L₂** über einem Katalysatorbett umfassend den entsprechenden Trägermetallkatalysator umgesetzt. Diese bevorzugte Ausführungsform hat den zusätzlichen Vorteil, dass ein Filtrationsschritt, in dem der Trägermetallkatalysator entfernt werden muss, entfällt.

2.2.2 Metallvollkatalysatoren sind dem Fachmann bekannt und beispielsweise in EP 3 266 766 A1 beschrieben. Ein "Metallvollkatalysator" ist ein vollständig von dem katalytischen Material durchdrungener Formkörper. Er unterscheidet sich damit vom "Trägermetallkatalysator", bei dem die katalytisch aktive Komponente auf einem von der katalytisch aktiven Komponente verschiedenen Träger aufgebracht ist.

Metallvollkatalysatoren weisen erfindungsgemäß mindestens ein elementares Metall Mᵥ auf, wobei das elementare Metall Mᵥ bevorzugt ausgewählt ist aus der Gruppe bestehend aus Ag, V, Cr, Mo, Mn, Ni, Pd, Pt, Fe, Ru, Os, Co, Rh, Ir, Cu; insbesondere ausgewählt ist aus der Gruppe bestehend aus Ag, Fe, Cr, Mo, Mn, Ni, Co, Cu, Pd, Pt, Ru, Rh; bevorzugt ausgewählt ist aus der Gruppe bestehend aus Ag, Fe, Cr, Ni, Co, Cu, Pd, Pt; bevorzugter ausgewählt ist aus der Gruppe bestehend aus Ag, Fe, Ni, Co, Cu, Pd, Pt; noch bevorzugter ausgewählt ist aus der Gruppe bestehend aus Ag, Fe, Ni, Co, Cu; noch mehr bevorzugter ausgewählt ist aus der Gruppe bestehend Co, Ni; am bevorzugtesten Ni ist.

Der Metallvollkatalysator kann eine Legierung des Metalls Mᵥ sein (wobei das Metall Mᵥ zu mindestens > 50 Gew.-% in der Legierung vorliegt, bezogen auf das Gesamtgewicht der Legierung) und beispielsweise außer Mᵥ noch mindestens ein Metall bzw. Halbmetall ausgewählt aus Al, Si, Mg, Zn, Mo, Cr, insbesondere Al, aufweisen.

Noch mehr bevorzugter weist der Metallvollkatalysator mindestens ein Metall Mᵥ ausgewählt aus der Gruppe bestehend aus Raney-Kobalt, Raney-Kupfer, Raney-Silber, Raney-Eisen, Raney-Nickel, insbesondere ausgewählt aus Raney-Nickel, Raney-Kobalt, am bevorzugtesten ausgewählt aus Raney-Nickel auf.

Die Synthese dieser Metallvollkatalysatoren sind dem Fachmann bekannt und beispielsweise in US 1,629,190, DE 20 2010 007837 U1 beschrieben. Dazu legiert man Ni mit Al, Si, Mg oder Zn (insbesondere mit Al, bevorzugt im Verhältnis 1:1) und löst aus der Legierung nach mechanischer Zerkleinerung mit Alkalien (insbesondere NaOH) das katalytisch unwirksame Metall (Al) mindestens teilweise heraus.

Entsprechend werden auch Raney-Kupfer, Raney-Kobalt, Raney Silber oder Raney-Eisen hergestellt (beschrieben zum Beispiel in der DE 20 2010 007837 U1).

Wird in Schritt (b) des erfindungsgemäßen Verfahrens H₂O₂ aus **L₂** mit einem Metallvollkatalysator entfernt, wird der Metallvollkatalysator zur wässrigen Lösung **L₂** zugegeben und H₂O₂ an diesem Metallvollkatalysator zu H₂O und O₂ disproportioniert.

Dabei wird die Temperatur in Schritt (b) bevorzugt bei 20 °C bis 80 °C, bevorzugter bei 40 °C bis 70 °C, noch bevorzugter bei 50 °C bis 60 °C, am bevorzugtesten bei 60 °C eingestellt.

Insbesondere beträgt das Gewicht (Massenanteil) des zugegebenen Vollkatalysators 0.1 % bis 5 %, bevorzugter 0.5 % bis 2 %, bezogen auf das Gesamtgewicht der wässrigen Lösung **L₂**. Bevorzugt, insbesondere bei kontinuierlicher Fahrweise des erfindungsgemäßen Verfahrens, wird bei Umsetzen der wässrigen Lösung **L₂** in Gegenwart eines Metallvollkatalysators in Schritt (b) die wässrige Lösung **L₂** über einem Katalysatorbett umfassend den entsprechenden Metallvollkatalysator umgesetzt. Diese bevorzugte Ausführungsform hat den zusätzlichen Vorteil, dass ein Filtrationsschritt, in dem der Metallvollkatalysator entfernt werden muss, entfällt.

2.2.3 Wird in Schritt (b) des erfindungsgemäßen Verfahrens H₂O₂ mit einem Metalloxidkatalysator entfernt, wird der Metalloxidkatalysator zur wässrigen Lösung **L₂** zugegeben und H₂O₂ an diesem Metallvollkatalysator zu H₂O und O₂ disproportioniert.

Dabei wird die Temperatur in Schritt (b) bevorzugt bei 20 °C bis 80 °C, bevorzugter bei 40 °C bis 70 °C, noch bevorzugter bei 50 °C bis 60 °C, am bevorzugtesten bei 60 °C eingestellt.

Insbesondere beträgt das Gewicht (Massenanteil) des zugegebenen Metalloxidkatalysators 0.1 % bis 5 %, bevorzugter 0.5 % bis 2 %, bezogen auf das Gesamtgewicht der wässrigen Lösung **L₂**. Bevorzugt, insbesondere bei kontinuierlicher Fahrweise des erfindungsgemäßen Verfahrens, wird bei Umsetzen der wässrigen Lösung **L₂** in Gegenwart eines Metalloxidkatalysators in Schritt (b) die wässrige Lösung **L₂** über einem Katalysatorbett umfassend den entsprechenden Metalloxidkatalysator umgesetzt. Diese bevorzugte Ausführungsform hat den zusätzlichen Vorteil, dass ein Filtrationsschritt, in dem der Metalloxidkatalysator entfernt werden muss, entfällt.

Der bevorzugteste Metalloxidkatalysator ist Braunstein, MnO₂.

2.2.4 Wird in Schritt (b) des erfindungsgemäßen Verfahrens H₂O₂ durch enzymatische Katalyse entfernt, wird das jeweilige Enzym zur wässrigen Lösung **L₂** zugegeben und H₂O₂ durch dieses zu H₂O und O₂ disproportioniert. Das Enzym wird bevorzugt auf einer festen Phase geträgert eingesetzt.

Als Enzym wird insbesondere bovine Katalase, wie beispielsweise von M. Ibrahim, H. G. Schlegel, Biotechnol Bioeng. 1980, 22, 1895-1906 beschrieben, genutzt.

Bevorzugt wird der Schritt (b) wie unter Punkt 2.2, bevorzugter wie unter Punkten 2.2.1 bis 2.2.3 beschrieben durchgeführt. Dies hat den Vorteil, dass bei der Entfernung des Wasserstoffperoxids keine zusätzlichen Salze eingetragen werden, wie es bei der Einstellung eines alkalischen pH-Wertes mit einer Base üblicherweise der Fall ist.

Von den Punkten 2.2.1 bis 2.2.3, sind die Verfahren wie unter Punkten 2.2.1 bis 2.2.2 beschrieben bevorzugter, noch bevorzugter das unter Punkt 2.2.1 beschriebene Verfahren.

Nach Durchführung von Schritt (a) bzw., wenn Schritt (b) durchgeführt wird, von Schritt (b) wird eine CO₂-haltige, wässrige Lösung **L₃** umfassend mindestens eine Verbindung der Formel **(I)** bzw. **(II)** erhalten.

Diese CO₂-haltige, wässrige Lösung **L₃** wird dann dem Schritt (c) des erfindungsgemäßen Verfahrens zugeführt.

In Schritt (b) des erfindungsgemäßen Verfahrens wird gegebenenfalls auch CO₂ teilweise aus **L₂** entfernt. Dies ist beispielsweise schon der Fall, wenn die wässrige Lösung **L₂** während dieses Schritts (b) insbesondere auf eine Temperatur von > 60 °C erhitzt wird, denn dann gast CO₂ teilweise aus.

Allerdings enthält auch in diesen Fällen die nach Schritt (b) erhaltene wässrige Lösung **L₃** CO₂, so dass eine Behandlung der wässrigen Lösung **L₃** gemäß Schritt (c) durchgeführt wird, um eine vollständige Entfernung von CO₂ zu gewährleisten.

### 3. Schritt (c)

In Schritt (c) des erfindungsgemäßen Verfahrens wird ein saurer pH-Wert in **L₃** eingestellt, wodurch CO₂ mindestens teilweise aus **L₃** entfernt und die wässrige Elektrolytlösung **L₁** erhalten wird.

In einer noch bevorzugteren Ausführungsform wird in **L₃** ein pH < 6.9, bevorzugt ein pH im Bereich von 1 bis 6, bevorzugter 2 bis 5, noch bevorzugter ein pH von 5 eingestellt.

Durch die Ansäuerung der wässrigen Lösung wird der Katalysator CO₂, der für die Umsetzung in Schritt (a) nötig war, ausgetrieben, und es wird eine wässrige Lösung **L₁** erhalten, welche in Ladungsspeichereinheiten verwendet werden kann. Diese hat einen gegenüber **L₃** verringerten Gehalt an CO₂.

In einer bevorzugten Ausführungsform wird in Schritt (c) der Anteil an CO₂ so verringert, dass die Summe der molaren Menge an gelöstem CO₂ in der wässrigen Lösung **L₁** < 10 Mol.-%, bevorzugt < 5 Mol.-%, bevorzugter < 1 Mol.-%, noch bevorzugter < 0.1 Mol.-% ist als der in der wässrigen Lösung **L₂** in Schritt (a).

Der saure pH-Wert in **L₃** wird praktischerweise eingestellt, in dem der wässrigen Lösung eine anorganische oder organische, bevorzugt eine anorganische Säure zugegeben wird. Als anorganische Säuren eignen sich insbesondere Salzsäure, Phosphorsäure, Schwefelsäure, Trifluormethansulfonsäure, Salpetersäure, Perchlorsäure, Borsäure, Blausäure, Flusssäure, am bevorzugtesten Salzsäure.

Es wird bevorzugt, dass in der wässrigen Elektrolytlösung **L₁** der Anteil aller Verbindungen der Formeln **(I)** und **(II)** bzw. wenn nur Verbindungen der Formeln **(III)** als Edukte eingesetzt werden, der Formel **(I)**, in der wässrige Lösung **L₁** im Bereich 10 bis 80 Gew.-%, bevorzugt 30 bis 70 Gew.-%, bezogen auf das Gesamtgewicht der wässrigen Elektrolytlösung **L₁** liegt (Massenanteil).

Die im erfindungsgemäßen Verfahren erhaltene wässrige Elektrolytlösung **L₁** kann in Ladungsspeichereinheiten, insbesondere Redox-flow-Batterien, eingesetzt werden.

Das erfindungsgemäße Verfahren stellt insbesondere gegenüber jenen des Standes der Technik ein überraschend einfaches Verfahren zur Herstellung von wässrigen Elektrolytlösungen von mindestens einer Verbindung der Formeln **(I)** und **(II)**, bevorzugt mindestens einer Verbindung der Formeln **(I)** dar. Sie zeichnet sich durch eine unkomplizierte Aufarbeitung der Reaktionslösungen auf und insbesondere dadurch, dass auf einen Filtrationsschritt verzichtet werden kann.

### 4. Edukte

Die im erfindungsgemäßen Verfahren eingesetzten Verbindungen der Formeln **(III)** und **(IV)** sind 2,2,6,6-Tetraalkylpiperidin-4-ammoniumsalze, deren Herstellung dem Fachmann geläufig sind. Deren Synthese ist beispielsweise in WO 2018/028830 A1 beschrieben.

In einer bevorzugten Ausführungsform des erfindungsgemäßen Verfahrens werden die eingesetzten Edukte der Formeln **(III)** und **(IV)** in einem vorhergehenden Schritt aus der Formel **(V)** erhalten mit:

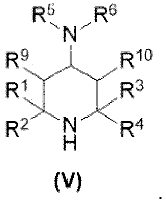

In der Formel (V) haben die Reste R¹, R², R³, R⁴, R⁵, R⁶, R⁹, R¹⁰ die vorgenannten Bedeutungen. Dazu wird die Verbindung mit der Formel **(V)** mit einem organischen Halogenid der Formel R⁷-Hal oder der Formel Hal-R⁸-(Hal)ₘ oder einem organischen Sulfat der Formel R⁷-O-SO₂-O-R⁷, worin R⁷, R⁸, m die vorgenannten Definitionen aufweisen, umgesetzt. Die Umsetzung mit dem genannten organischen Halogenid R⁷-Hal ist dabei bevorzugt. Hal ist dabei aus der Gruppe bestehend aus F, Cl, Br, I ausgewählt, bevorzugt aus der Gruppe bestehend aus Cl, Br, I und am bevorzugtesten Cl.

Diese Umsetzung läuft in einem Lösungsmittel ab. Dabei handelt es sich dabei bevorzugt um ein organisches Lösungsmittel, welches aus der Gruppe bestehend aus Ether, Nitril, halogenierter Kohlenwasserstoff, aromatischer Kohlenwasserstoff, aliphatischer Kohlenwasserstoff ausgewählt ist.

Bevorzugte Ether sind Diethylether und Di-isopropylether. Beispiele für Nitrile sind Acetonitril.

Bevorzugte halogenierte Kohlenwasserstoffe sind chlorierte und/oder fluorierte aliphatische Kohlenwasserstoffe, wie Dichlormethan, Chloroform, Tetrachlorkohlenstoff, Dichlorethan oder Chlorfluorethan.

Bevorzugte aromatische Kohlenwasserstoffe sind Benzol, Toluol oder Xylol. Beispiele für aliphatische Kohlenwasserstoffe sind Hexan, Octan oder Decan sowie Petrolether oder Petroleum.

Als R⁷-Hal werden bevorzugt Alkylchloride, Alkylbromide oder Alkyljodide eingesetzt, besonders bevorzugt aber Alkylchloride. Bevorzugte Beispiele für Alkylchloride der Formel R⁷-Cl sind Methylchlorid, Ethylchlorid. Ganz besonders bevorzugt ist Methylchlorid.

Beispiele dafür sind Alkylchloride, deren Alkylgruppe ein bis sechs Kohlenstoffatome aufweist. Bevorzugt befindet sich das Chloratom am endständigen Kohlenstoffatom.

Als Hal-R⁸-(Hal)ₘ eingesetzte Chloride weisen zwei bis sechs Chloratome auf, bevorzugt werden Alkyldi-, Alkyltri-, Alkylquater-, Alkylpenta- und Alkylhexachloride. Beispiele dafür sind Alkyldichloride oder Alkyltrichloride, deren Alkylengruppen oder Alkyltriylgruppen ein bis sechs Kohlenstoffatome aufweisen. Am bevorzugtesten ist 1,6-Dichlorhexan, 1,4-Dichlorbutan und Ethylendichlorid (EDC).

Bei R⁷-O-SO₂-O-R⁷ handelt es sich um ein organisches Sulfat. Bevorzugt werden Dialkylsulfate, insbesondere Dimethylsulfat.

Das molare Verhältnis von 4-Amino-alkylpiperidin der Formel **(V)** zu Alkylchlorid der Formel R⁷-Hal in der Umsetzung beträgt üblicherweise 1 : 5 bis 1 : 1 , insbesondere 1 : 3 bis 1 : 1 und ganz besonders bevorzugt 1 : 1.05 bis 1 : 1.

Das molare Verhältnis von 4-Amino-alkylpiperidin der Formel **(V)** zu organischem Chlorid der Formel Hal-R⁸-(Hal)ₘ beträgt üblicherweise 1 : 5 × m bis 1 : m, insbesondere 1 : 3 × m bis 1 : m und ganz besonders bevorzugt 1 : 2 × m bis 1 : m, worin m die oben definierte Bedeutung besitzt.

Das molare Verhältnis von 4-Amino-alkylpiperidin der Formel **(V)** zu organischem Sulfat der Formel R⁷-O-SO₂-O-R⁷ beträgt üblicherweise 1 : 5 bis 1 : 1 , insbesondere 1 : 3 bis 1 : 1 und ganz besonders bevorzugt 1 : 2 bis 1 : 1.

Die Umsetzung von Verbindungen der Formel **(V)** zu Verbindungen der Formel **(III)** oder **(IV)** erfolgt insbesondere bei Temperaturen im Bereich von 0 °C bis 200 °C, vorzugsweise von 25 °C bis 150 °C, und ganz besonders bevorzugt bei 40 °C bis 100 °C. Bevorzugte Drucke liegen dabei im Bereich von 1 bar bis 250 bar, vorzugsweise von 1 bar bis 100 bar, und ganz besonders bevorzugt bei 1 bar bis 6 bar. Die Umsetzung kann in An- und Abwesenheit einer Base durchgeführt werden. Insbesondere bei 4-Amino-alkylpiperidinylverbindungen der Formel **(V),** in denen die 4-Aminogruppe ein oder mehrere Wasserstoffatome aufweist, ist es bevorzugt, wenn die Umsetzung in Anwesenheit einer Base durchgeführt wird. Beispiele für geeignete Basen sind anionische lonenaustauscher, Alkalihydroxide, wie Natriumhydroxid oder Kaliumhydroxid, oder organische Amine, vorzugsweise solche Amine, die bei den Drucken und Temperaturen in der Umsetzung als flüssige Phase vorliegen.

Die Verbindungen der Formel **(V)** wiederum können nach dem Fachmann bekannten Verfahren, wie sie beispielsweise in der WO 2018/028830 A1 beschrieben sind, aus den entsprechenden 4-Oxo-Alkylpiperidinverbindungen oder den entsprechenden Iminen hergestellt werden.

### Allgemeine Begriffe

R⁸ ist eine p-wertige organische Brückengruppe, wobei p eine ganze Zahle zwischen zwei und sechs ist. Darunter ist demnach ein organischer Rest zu verstehen, der über zwei, drei, vier, fünf oder sechs kovalente Bindungen mit dem Rest des Moleküls verbunden ist. R⁸ ist bevorzugt Alkylen.

Beispiele für zweiwertige organische Brückengruppen sind Alkylen, Alkylenoxy, Poly(alkylenoxy), Alkylenamino, Poly(alkylenamino), Cycloalkylen, Arylen, Aralkylen oder Heterocyclylen.

Alkylengruppen können sowohl verzweigt als auch unverzweigt sein. Eine Alkylengruppe enthält typischerweise ein bis zu zwanzig Kohlenstoffatome, bevorzugt zwei bis zu sechs Kohlenstoffatome. Beispiele für Alkylengruppen sind: Methylen, Ethylen, Propylen und Butylen, Pentylen, Hexylen. Alkylengruppen können gegebenenfalls substituiert sein, beispielsweise mit Alkyl, Alkoxy, Hydroxy, Carboxyl, Carboxylester, Carboxylamid, Sulfonat, Sulfonsäureester, Sulfonamid, Halogen.

Alkylenoxy- und Poly(alkylenoxy)gruppen können sowohl verzweigte als auch unverzweigte Alkylengruppen enthalten. Eine in einer Alkylenoxy- oder in einer Poly(alkylenoxy)gruppe auftretende Alkylengruppe enthält typischerweise zwei bis vier Kohlenstoffatome, bevorzugt zwei oder drei Kohlenstoffatome. Die Anzahl der Wiederholeinheiten in den Poly(alkylenoxy)gruppen kann in weiten Bereichen schwanken. Typische Anzahlen von Wiederholeinheiten bewegen sich im Bereich von 2 bis 50. Beispiele für Alkylenoxygruppen sind: Ethylenoxy, Propylenoxy und Butylenoxy. Beispiele für Poly(alkylenoxy)gruppen sind: Poly(ethylenoxy), Poly(propylenoxy) und Poly(butylenoxy).

Alkylenamino- und Poly(alkylenamino)gruppen können sowohl verzweigte als auch unverzweigte Alkylengruppen enthalten. Eine in einer Alkylenamino- oder in einer Poly(alkylenamino)gruppe auftretende Alkylengruppe enthält typischerweise zwei bis vier Kohlenstoffatome, bevorzugt zwei oder drei Kohlenstoffatome. Die Anzahl der Wiederholeinheiten in den Poly(alkylenamino)gruppen kann in weiten Bereichen schwanken. Typische Anzahlen von Wiederholeinheiten bewegen sich im Bereich von 2 bis 50. Beispiele für Alkylenaminogruppen sind: Ethylenamino, Propylenamino und Butylenamino. Beispiele für Poly(alkylenamino)gruppen sind: Poly(ethylenamino), Poly(propylenamino) und Poly(butylenamino).

Cycloalkylengruppen enthalten typischerweise fünf, sechs oder sieben Ringkohlenstoffatome, die jeweils unabhängig voneinander substituiert sein können. Beispiele für Substituenten sind Alkylgruppen oder zwei Alkylgruppen, die zusammen mit den Ringkohlenstoffen, an denen sie gebunden sind, einen weiteren Ring bilden können. Ein Beispiel für eine Cycloalkylengruppe ist Cyclohexylen.

Arylengruppen sind typischerweise cyclische aromatische Gruppen, die fünf bis vierzehn Kohlenstoffatome enthalten, die jeweils unabhängig voneinander substituiert sein können. Beispiele für Arylengruppen sind o-Phenylen, m-Phenylen, p-Phenyl, Naphthylengruppen oder Biphenylengruppen.

Heterocyclylgruppen sind typischerweise cyclische Gruppen mit vier bis zehn Ringkohlenstoffatomen und mindestens einem Ringheteroatom, die jeweils unabhängig voneinander substituiert sein können, insbesondere mit Alkyl. Beispiele für Heteroatome sind Sauerstoff, Stickstoff, Phosphor, Bor, Selen oder Schwefel. Beispiele für Heterocyclylengruppen sind Furandiyl, Thiophendiyl, Pyrroldiyl oder Imidazoldiyl. Heterocyclylengruppen sind vorzugsweise aromatisch.

Aralkylengruppen sind typischerweise Arylgruppen, an die ein oder zwei Alkylgruppen kovalent gebunden sind. Aralkylgruppen können über ihren Arylrest und ihren Alkylrest oder über zwei Alkylreste mit dem Rest des Moleküls kovalent verbunden sein. Die Aralkylengruppe kann am aromatischen Ring beispielsweise mit Alkylgruppen oder mit Halogenatomen substituiert sein. Beispiele für Aralkylengruppen sind Benzylen oder Dimethylphenylen (Xylylen).

Beispiele für dreiwertige organische Reste R⁸ sind Alkyltriyl, Alkoxytriyl, Trispoly(alkylenoxy), Tris-poly(alkylenamino), Cycloalkyltriyl, Aryltriyl, Aralkyltriyl oder Heterocyclyltriyl. Diese Reste entsprechen den bereits weiter oben eingehend beschriebenen zweiwertigen Resten mit dem Unterschied, dass diese mit drei kovalenten Bindungen anstelle von zwei kovalenten Bindungen mit dem Rest des Moleküls verbunden sind.

Beispiele für vierwertige organische Reste R⁸ sind Alkylquaternyl, Alkoxyquaternyl, Quaterpoly(alkylenoxy), Quaterpoly(alkylenamino), Cycloalkylquaternyl, Arylquaternyl, Aralkylquaternyl oder Heterocyclylquaternyl. Diese Reste entsprechen den bereits weiter oben eingehend beschriebenen zweiwertigen Resten mit dem Unterschied, dass diese mit vier kovalenten Bindungen anstelle von zwei kovalenten Bindungen mit dem Rest des Moleküls verbunden sind.

Beispiele für fünfwertige organische Reste R⁸ sind Alkylquinquinyl, Alkoxyquinquinyl, Quinquipoly(alkylenoxy), Quinquipoly(alkylenamino), Cycloalkylquinquinyl, Arylquinquinyl, Aralkylquinquinyl oder Heterocyclylquinquinyl. Diese Reste entsprechen den bereits weiter oben eingehend beschriebenen zweiwertigen Resten mit dem Unterschied, dass diese mit fünf kovalenten Bindungen anstelle von zwei kovalenten Bindungen mit dem Rest des Moleküls verbunden sind.

Beispiele für sechswertige organische Reste R⁸ sind Alkylhexyl, Alkoxyhexyl, Hexylpoly(alkylenoxy), Hexylpoly(alkylenamino), Cycloalkylhexyl, Arylhexyl, Aralkylhexyl oder Heterocyclylhexyl. Diese Reste entsprechen den bereits weiter oben eingehend beschriebenen zweiwertigen Resten mit dem Unterschied, dass diese mit sechs kovalenten Bindungen anstelle von zwei kovalenten Bindungen mit dem Rest des Moleküls verbunden sind.

Beispiele für anorganische Anionen X^{a-} sind Halogenidionen, wie Fluorid, Chlorid, Bromid oder Jodid, oder Hydroxidionen oder Anionen anorganischer Säuren, wie Phosphat, Sulfat, Nitrat, Borat, Hexafluorophosphat, Tetrafluoroborat, Perchlorat, Chlorat, Hexafluoroantimonat, Hexafluoroarsenat, Cyanid. Zu den Sulfat- oder Phospatanionen zählen auch solche, die einen organischen Rest aufweisen, insbesondere einen Alkylrest, ganz besonders bevorzugt einen Methylrest. Bevorzugt wird das Methylsulfatanion CH₃-OSO₃⁻.

Beispiele für organische Anionen X^{a-} sind Anionen ein- oder mehrwertiger Carbonsäuren oder ein- oder mehrwertiger Sulfonsäuren, wobei diese Säuren gesättigt oder ungesättigt sein können. Weitere Beispiele für organische Anionen X^{a-} sind Anionen von Polycarbonsäuren oder von Polysulfonsäuren. Beispiele für Anionen organischer Säuren sind Acetat, Formiat, Trifluoroacetat, Trifluormethansulfonat, Pentafluorethansulfonat, Nonofluorbutansulfonat, Butyrat, Citrat, Fumarat, Glutarat, Lactat, Malat, Malonat, Oxalat, Pyruvat oder Tartrat. Beispiele für Anionen von Polycarbonsäuren sind Polyacrylat- oder Polymethacrylatanionen. Ein Beispiel für Anionen von Polysulfonsäuren ist Polystyrensulfonat (PSS).

Bedeutet einer der Reste R⁵, R⁶, R⁵', R⁶' und/oder R⁷ Alkyl, so kann die Alkylgruppe sowohl verzweigt als auch unverzweigt sein. Eine Alkylgruppe dieser Reste enthält typischerweise ein bis zu zwanzig Kohlenstoffatome, bevorzugt ein bis zu zehn Kohlenstoffatome. Beispiele für Alkylgruppen sind: Methyl, Ethyl, *n*-Propyl, *iso*-Propyl, *n*-Butyl, *sec*-Butyl, *tert*-Butyl, Pentyl, *n*-Hexyl, *n*-Heptyl, 2-Ethylhexyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*- Hexadecyl, *n*-Heptadecyl, *n*-Octadecyl, *n*-Nonadecyl oder Eicosyl. Besonders bevorzugt sind von den vorgenannten Alkylgruppen jene mit ein bis sechs, noch bevorzugter jene mit ein bis vier Kohlenstoffatomen.

Die Reste R⁵, R⁶, R⁵', R⁶' und/oder R⁷ als Alkyl können gegebenenfalls substituiert sein, beispielsweise mit Alkoxy, Hydroxy, Carboxyl, Carboxylester, Carboxylamid, Sulfonat, Sulfonsäureester, Sulfonamid oder Halogen, bevorzugt mit Alkoxy, Hydroxy, Halogen. Der Rest R⁷ als Alkyl kann gegebenenfalls auch mit Nitro substituiert sein.

Die Reste R⁵, R⁶, R⁵', R⁶' und/oder R⁷ als Alkyl können gegebenenfalls durch ein oder mehrere, nicht direkt zueinander benachbarte zweiwertige Sauerstoff- oder Iminoreste unterbrochen sein. Beispiele dafür sind einwertige Reste abgeleitet von Polyethylenglykolen, von Polypropylenglykolen, von Polyethyleniminen oder von Polypropyleniminen.

Bedeutet einer der Reste R⁵, R⁶, R⁵' und/oder R⁶' Cycloalkyl, so ist die Cycloalkylgruppe typischerweise eine cyclische Gruppe, enthaltend fünf bis acht, vorzugsweise fünf, sechs oder sieben Ringkohlenstoffatome, die jeweils unabhängig voneinander substituiert sein können. Beispiele für Substituenten sind Alkylgruppen oder zwei Alkylgruppen, die zusammen mit den Ringkohlenstoffen, an denen sie gebunden sind, einen weiteren Ring bilden können. Beispiele für Cycloalkylgruppen sind Cyclopentyl oder Cyclohexyl. Cycloalkylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁵' und/oder R⁶' Aryl, so ist die Arylgruppe typischerweise eine cyclische aromatische Gruppe, enthaltend fünf bis vierzehn Kohlenstoffatome, die jeweils unabhängig voneinander substituiert sein können. Beispiele für Substituenten sind Alkylgruppen oder zwei Alkylgruppen, die gemeinsam mit den Ringkohlenstoffatomen, an denen sie gebunden sind, einen weiteren Ring bilden können. Beispiele für Arylgruppen sind Phenyl, Biphenyl, Anthryl oder Phenantolyl. Arylgruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁵' und/oder R⁶' Heterocyclyl, so kann die Heterocyclylgruppe typischerweise eine cyclische Gruppe mit vier bis zehn Ringkohlenstoffatomen und mindestens einem Ringheteroatom aufweisen, die jeweils unabhängig voneinander substituiert sein können.

Beispiele für Substituenten der Heterocyclylgruppe sind Alkylgruppen oder zwei Alkylgruppen die zusammen mit den Ringkohlenstoffen an denen sie gebunden sind einen weiteren Ring bilden können. Beispiele für Heteroatome sind Sauerstoff, Stickstoff, Phosphor, Bor, Selen oder Schwefel. Beispiele für Heterocyclyigruppen sind Furyl, Thienyl, Pyrrolyl oder Imidazolyl. Heterocyclyigruppen sind vorzugsweise aromatisch. Heterocyclyigruppen können gegebenenfalls mit Alkyl, Alkoxy, Hydroxy, Carboxyl, Carboxylester, Carboxylamid, Sulfonat, Sulfonsäureester, Sulfonamid oder Halogen substituiert sein.

Bedeutet einer der Reste R⁵, R⁶, R⁵' und/oder R⁶' Aralkyl, so ist die Aralkylgruppe typischerweise eine Arylgruppe, wobei Aryl zuvor bereits definiert wurde, an die kovalent eine Alkylgruppe gebunden ist. Die Aralkylgruppe kann am aromatischen Ring beispielsweise mit Alkylgruppen oder mit Halogenatomen substituiert sein. Ein Beispiel für eine Aralkylgruppe ist Benzyl. Aralkylgruppen können gegebenenfalls mit Alkoxy, Hydroxy, Carboxyl, Carboxylester, Carboxylamid, Sulfonat, Sulfonsäureester, Sulfonamid oder Halogen, bevorzugt mit Alkoxy, Hydroxy, Halogen substituiert sein.

In einer bevorzugten Ausführungsform der vorliegenden Erfindung sind R⁵, R⁶, R⁵', R⁶' unabhängig voneinander jeweils Alkyl, welches gegebenenfalls mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid oder Halogen substituiert sind und/oder die gegebenenfalls in der Alkylgruppe durch ein oder mehrere, nicht direkt zueinander benachbarte zweiwertige Reste ausgewählt aus der Gruppe bestehend aus Sauerstoff, Imino unterbrochen sind.

In einer weiteren bevorzugten Ausführungsform der vorliegenden Erfindung ist R⁷ Alkyl, das gegebenenfalls mit ein oder mehreren Resten ausgewählt aus der Gruppe bestehend aus Alkoxy, Hydroxy, Nitro, Carboxyl, Carboxylester, Carboxylamid, Sulfonat, Sulfonsäureester, Sulfonamid oder Halogen substituiert ist und/oder das gegebenenfalls in der Alkylgruppe durch ein oder mehrere, nicht direkt zueinander benachbarte zweiwertige Reste ausgewählt aus der Gruppe bestehend aus Sauerstoff, Imino unterbrochen ist.

Ganz besonders bevorzugt bedeuten R⁵, R⁶, R⁵', R⁶' und/oder R⁷ unabhängig voneinander C₁-C₆-Alkyl.

Bedeutet einer der Substituenten Halogen, so ist darunter ein kovalent gebundenes Fluor-, Chlor-, Brom- oder Jodatom zu verstehen. Bevorzugt ist als Halogen Chlor oder Brom.

### Beispiele

### Erfinderische Beispiele 1 bis 4

Ein Mehrhalskolben wurde mit 149.4 g entionisiertem Wasser beladen und auf 60 °C erhitzt. Unter Rühren wurden schließlich 234.8 g (1 mol) N,N,N,2,2,6,6-Heptamethylpiperidinyl-4-ammoniumchlorid (abgekürzt als "TMA-TEMP") zugegeben und gelöst. Anschließend wurden 4.4 g (0.1 mol) festes Kohlenstoffdioxid (Trockeneis) zugegeben. Zum Schluss wurden 102 g H₂O₂ als 50 Gew.-% wässrige Lösung (1.5 mol H₂O₂) zugegeben. Bezogen auf TMA-TEMP wurden demnach 1.5 molare Äquivalente H₂O₂ eingesetzt. Die Zugabe des H₂O₂ zur Reaktionsmischung erfolgte in einer Geschwindigkeit, dass die Temperatur der Mischung nicht über 60 °C stieg. Nach beendeter Zugabe des H₂O₂ wurde die Reaktionsmischung für eine Stunde bei 60 °C gerührt.

Die Aufarbeitung (Entfernung des überschüssigen H₂O₂) erfolgte wie in Beispiel 1-5 beschrieben.

Beispiel 1: Die Reaktionslösung wurde auf 80 °C erhitzt und 50 Gew.-%ige wässrige Natriumhydroxidlösung zugegeben, um einen pH von 10.6 bis 11 einzustellen.

Beispiel 2: 2.5 g Platin auf Aluminiumoxid wurden bei 60 °C zugegeben und für 30 Minuten bei 60 °C gerührt. Überschüssiges H₂O₂ wurde so zerstört. Eine wässrige Lösung von N,N,N,2,2,6,6-Heptamethylpiperidinyl-1-oxy-4-ammoniumchlorid (abgekürzt als "TMA-TEMPO") wurde durch Filtration erhalten.

Beispiel 3: 2.5 g Palladium auf Aluminiumoxid wurden bei 60 °C zugegeben und für 30 Minuten bei 60 °C gerührt. Überschüssiges H₂O₂ wurde so zerstört. Eine wässrige Lösung von TMA-TEMPO wurde durch Filtration erhalten.

Beispiel 4: 2.5 g Mangandioxid wurden bei 60 °C zugegeben und für 30 Minuten bei 60 °C gerührt. Überschüssiges H₂O₂ wurde so zerstört. Eine wässrige Lösung von TMA-TEMPO wurde durch Filtration erhalten.

Beispiel 5: Die Reaktionsmischung wurde in einem Reaktionsrohr über 2.5 g Platin auf Aluminiumoxid geleitet. Überschüssiges H₂O₂ wurde so zerstört. Eine wässrige Lösung von N,N,N,2,2,6,6-Heptamethylpiperidinyl-1-oxy-4-ammoniumchlorid (abgekürzt als "TMA-TEMPO") wurde so erhalten.

Nach Zerstörung von H₂O₂ wurde die jeweilige Reaktionslösung durch langsame Zugabe von 37 Gew.-% HCl auf pH 5 eingestellt, um CO₂ weiter auszutreiben.

Im Vergleich zum Stand der Technik (WO 2018/028830 A1; Beispiel Seite 29, Zeilen 4 bis 14) ergeben sich durch das erfindungsgemäße Verfahren überraschende Vorteile:
1) Durch die Vorgehensweise gemäß Beispielen 2 bis 5 wird bei der Entfernung des H₂O₂ kein zusätzliches Salz in die Lösung eingetragen, da der Katalysator zur Zerstörung des H₂O₂ heterogen eingesetzt wird. Dadurch wird die Salzfracht in der erhaltenen Lösung minimiert und somit die Löslichkeit der Zielverbindungen in der Elektrolytlösung erhöht, was sie besonders geeignet für den Einsatz in Ladungsspeichereinheiten macht.
2) Es werden lediglich 1.5 Äquivalente H₂O₂ eingesetzt, während im Stand der Technik zur Umsetzung von 383 mmol TMA-TEMP 1.15 mol H₂O₂ benötigt werden, also 3 Äquivalente. Das erfindungsgemäße Verfahren ist demnach deutlich effizienter.
3) Eine aufwendige Aufarbeitung wie Fällung und Filtration des Reaktionsprodukts TMA-TEMPO ist nicht nötig. Es muss lediglich der eingesetzte heterogene Katalysator abfiltriert werden, was technisch weniger aufwendig ist als die Filtration eines Niederschlags. Daneben kann die Reaktion auch kontinuierlich in einem mit heterogenem Katalysator gefüllten Zersetzerrohr durchgeführt werden, womit die Filtration vollends entfällt.

## Patentansprüche

1. Verfahren zur Herstellung einer wässrigen Elektrolytlösung **L₁** umfassend mindestens eine Verbindung der Formel **(I)** oder **(II)** mit 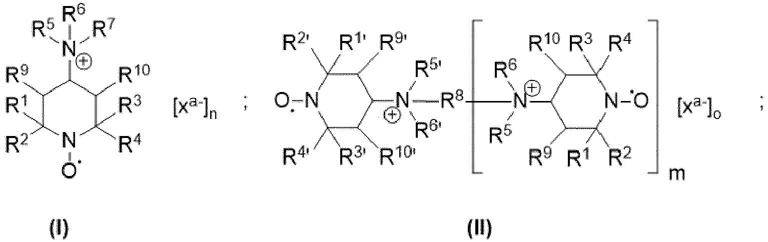
wobei R¹, R², R³, R⁴, R¹', R²', R³', R⁴' unabhängig voneinander jeweils ein C₁-C₆-Alkylrest sind,
wobei R⁹, R¹⁰, R⁹', R¹⁰' unabhängig voneinander jeweils aus der Gruppe bestehend aus Wasserstoff, C₁-C₆-Alkylrest ausgewählt sind,
wobei R⁵, R⁶, R⁵', R⁶' unabhängig voneinander jeweils aus der Gruppe bestehend aus Wasserstoff, Alkyl, Cycloalkyl, Aryl, Aralkyl, Heterocyclyl ausgewählt sind, wobei die Reste Alkyl, Cycloalkyl, Aryl, Aralkyl, Heterocyclyl mit ein oder mehreren Resten ausgewählt aus der Gruppe bestehend aus Alkoxy, Hydroxy, Carboxyl, Carboxylester, Carboxylamid, Sulfonat, Sulfonsäureester, Sulfonamid, Halogen substituiert sein können, wobei außerdem Alkyl durch ein oder mehrere, nicht direkt zueinander benachbarte zweiwertige Reste ausgewählt aus der Gruppe bestehend aus Sauerstoff, Imino unterbrochen sein kann,
wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei außerdem R⁵' und R⁶' zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei R⁷ ein Alkylrest ist, der mit ein oder mehreren Resten ausgewählt aus der Gruppe bestehend aus Alkoxy, Hydroxy, Nitro, Carboxyl, Carboxylester, Carboxylamid, Sulfonat, Sulfonsäureester, Sulfonamid, Halogen substituiert sein kann und der durch ein oder mehrere, nicht direkt zueinander benachbarte zweiwertige Reste ausgewählt aus der Gruppe bestehend aus Sauerstoff, Imino unterbrochen sein kann,
wobei **R⁸** eine p-wertige organische Brückengruppe ist, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann, und die durch ein oder mehrere, nicht direkt zueinander benachbarte zweiwertige Reste ausgewählt aus der Gruppe bestehend aus Sauerstoff, Imino unterbrochen sein kann,
X^{a-} ein organisches Anion, ein anorganisches Anion oder eine Mischung dieser Anionen ist,
a die Wertigkeit des Anions X^{a-} angibt und eine ganze Zahl von 1 bis 10.000 bedeutet,
p eine ganze Zahl von 2 bis 6 bedeutet,
m eine ganze Zahl mit dem Wert "p - 1" bedeutet,
n eine Zahl mit dem Wert "1 / a" bedeutet,
o eine Zahl mit dem Wert "(1 + m) / a" bedeutet,
**gekennzeichnet dadurch, dass**
(a) mindestens eine Verbindung der Formel (III) oder (IV) mit 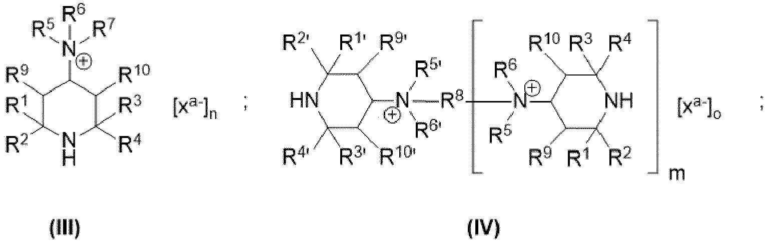 mit H₂O₂ in einer CO₂-haltigen, wässrigen Lösung **L₂** zu einer Verbindung der Formel **(I)** bzw. **(II)** umgesetzt wird und
(b) nach der Umsetzung gemäß Schritt (a) H₂O₂, falls dieses dann noch in der wässrigen Lösung **L₂** enthalten ist, aus **L₂** entfernt wird, und gegebenenfalls auch CO₂ teilweise aus **L₂** entfernt wird,
wodurch eine CO₂-haltige, wässrige Lösung **L₃** umfassend mindestens eine Verbindung der Formel **(I)** bzw. **(II)** erhalten wird,
(c) ein saurer pH-Wert in **L₃** eingestellt wird, wodurch CO₂ mindestens teilweise aus **L₃** entfernt und die wässrige Elektrolytlösung **L₁** erhalten wird.

2. Verfahren nach Anspruch 1,
wobei R¹, R², R³, R⁴, R¹', R²', R³', R⁴' unabhängig voneinander jeweils ein C₁-C₄-Alkylrest sind,
wobei R⁵, R⁶, R⁵', R⁶' unabhängig voneinander jeweils aus der Gruppe bestehend aus Wasserstoff, Alkyl, wobei Alkyl durch ein oder mehrere, nicht direkt zueinander benachbarte zweiwertige Reste ausgewählt aus der Gruppe bestehend aus Sauerstoff, Imino unterbrochen sein kann, ausgewählt sind,
wobei außerdem R⁵ und R⁶ zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei außerdem R⁵' und R⁶' zusammen eine Alkylengruppe, die durch ein oder mehrere, nicht direkt zueinander benachbarte zweiwertige Reste ausgewählt aus der Gruppe bestehend aus Sauerstoff, Imino unterbrochen sein kann, bilden können,
wobei R⁹, R¹⁰, R⁹', R¹⁰' unabhängig voneinander jeweils aus der Gruppe bestehend aus Wasserstoff, C₁-C₄-Alkylrest ausgewählt sind,
wobei R⁷ C₁-C₆-Alkyl, welches durch ein oder mehrere, nicht direkt zueinander benachbarte zweiwertige Reste ausgewählt aus der Gruppe bestehend aus Sauerstoff, Imino unterbrochen sein kann, ist,
wobei R⁸ eine Alkylengruppe, die mit ein oder mehreren Resten ausgewählt aus der Gruppe bestehend aus Alkyl, Hydroxy, Nitro, Carboxyl, Carboxylester, Carboxylamid, Sulfonat, Sulfonsäureester, Sulfonamid, Halogen substituiert sein kann und die durch ein oder mehrere, nicht direkt zueinander benachbarte zweiwertige Reste ausgewählt aus der Gruppe bestehend aus Sauerstoff, Imino unterbrochen sein kann, ist,
X^{a-} ein anorganisches Anion ist,
a bei Verbindungen der Formel **(I)** und **(III)** eine ganze Zahl von 1 bis 3 und bei Verbindungen der Formel **(II)** und **(IV)** eine ganze Zahl von 1 bis 6 bedeutet,
m = 1 bedeutet
n eine Zahl mit dem Wert "1 / a" bedeutet,
o eine Zahl mit dem Wert "2 / a" bedeutet.

3. Verfahren nach Anspruch 2, wobei R¹, R², R³, R⁴, R¹', R²', R³', R⁴' jeweils Methyl sind,
wobei R⁵, R⁶, R⁵', R⁶' unabhängig voneinander jeweils aus der Gruppe bestehend aus Methyl, Ethyl ausgewählt sind,
wobei R⁹, R¹⁰, R⁹', R¹⁰' jeweils Wasserstoff sind,
wobei R⁷ aus der Gruppe bestehend aus Methy, Ethyl ausgewählt ist,
wobei R⁸ eine C₁-C₁₀-Alkylengruppe ist,
X^{a-} aus der Gruppe bestehend aus Halogenidion mit Wertigkeit a = 1, Hydroxyion mit Wertigkeit a = 1, Phosphation mit Wertigkeit a = 3, Sulfation mit Wertigkeit a = 2, Perchloration mit Wertigkeit a = 1, Hexafluorophosphation mit Wertigkeit a = 1, Tetrafluoroboration mit Wertigkeit a = 1 ausgewählt ist,
m = 1 bedeutet,
n eine Zahl mit dem Wert "1 / a" bedeutet,
o eine Zahl mit dem Wert "2 / a" bedeutet.

4. Verfahren nach einem der Ansprüche 1 bis 3 zur Herstellung einer wässrigen Lösung **L₁** mindestens einer Verbindung der Formel **(I)**, wobei mindestens eine Verbindung der Formel **(III)** eingesetzt wird und die Definitionen der Reste R¹, R², R³, R⁴, R⁵, R⁶, R⁷, R⁹, R¹⁰, X^{a-} und der Variablen n nach einem der Ansprüche 1 bis 3 entsprechend gelten.

5. Verfahren nach einem der Ansprüche 1 bis 4, wobei Schritt (a) bei einem von pH-Wert von 7.5 bis < 10.5 durchgeführt wird.

6. Verfahren nach einem der Ansprüche 1 bis 5, wobei Schritt (a) bei einer Temperatur von 20 °C bis 100 °C und einem Druck von 0.5 bar bis 2 bar durchgeführt wird.

7. Verfahren nach einem der Ansprüche 1 bis 6, wobei vor Schritt (a) CO₂ in Form von Trockeneis und/oder gasförmig in Wasser gelöst wird, wodurch eine CO₂-haltige, wässrige Lösung **L₂** erhalten wird.

8. Verfahren nach einem der Ansprüche 1 bis 7, wobei das molare Verhältnis der Stoffmenge des in der wässrigen Lösung **L₂** gelösten CO₂ zur gesamten Stoffmenge aller in Schritt (a) eingesetzten Verbindungen der Formeln **(III)** und **(IV)** im Bereich 10 : 1 bis 1 : 1000 liegt.

9. Verfahren nach einem der Ansprüche 1 bis 8, wobei das molare Verhältnis des Gesamtgewichts aller in Schritt (a) eingesetzten Verbindungen der Formeln **(III)** und **(IV)** zum Gesamtgewicht des in Schritt (a) eingesetzten H₂O₂ im Bereich 9 : 1 bis 1 : 9 liegt.

10. Verfahren nach Anspruch 9, wobei das molare Verhältnis des Gesamtgewichts aller in Schritt (a) eingesetzten Verbindungen der Formeln **(III)** und **(IV)** zum Gesamtgewicht des in Schritt (a) eingesetzten H₂O₂ im Bereich 1 : 1.05 bis 1 : 9 liegt.

11. Verfahren nach Anspruch 10, wobei in Schritt (b) H₂O₂ durch Einstellen der wässrigen Lösung **L₂** auf einen pH-Wert von ≥ 10.5 entfernt wird.

12. Verfahren nach Anspruch 10, wobei in Schritt (b) H₂O₂ durch Umsetzen der wässrigen Lösung **L₂** in Gegenwart eines Katalysators entfernt wird.

13. Verfahren nach einem der Ansprüche 1 bis 12, wobei der Anteil aller Verbindungen der Formel **(I)** und **(II)** in der wässrigen Elektrolytlösung **L₁** im Bereich 10 bis 80 Gew.-%, bezogen auf das Gesamtgewicht der der wässrigen Elektrolytlösung **L₁**, liegt.

## Claims

1. Process for preparing an aqueous electrolyte solution L₁ comprising at least one compound of formula (I) or (II) 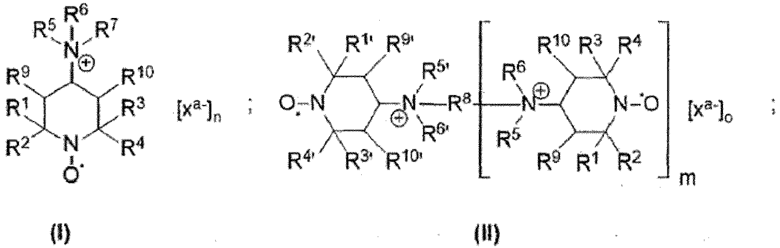
where R¹, R², R³, R⁴, R¹', R²', R³', R⁴' are each independently a C₁-C₆ alkyl radical,
where R⁹, R¹⁰, R⁹', R¹⁰' are each independently selected from the group consisting of hydrogen, C₁-C₆ alkyl radical,
where R⁵, R⁶, R⁵', R⁶' are each independently selected from the group consisting of hydrogen, alkyl, cycloalkyl, aryl, aralkyl, heterocyclyl, where the radicals alkyl, cycloalkyl, aryl, aralkyl, heterocyclyl may be substituted by one or more radicals selected from the group consisting of alkoxy, hydroxy, carboxyl, carboxylic ester, carboxamide, sulfonate, sulfonic ester, sulfonamide, halogen, where in addition alkyl may be interrupted by one or more divalent radicals that are not directly adjacent to one another and are selected from the group consisting of oxygen, imino,
where in addition R⁵ and R⁶ may together form an alkylene group that may be interrupted by one or more divalent radicals that are not directly adjacent to one another and are selected from the group consisting of oxygen, imino,
where in addition R⁵' and R⁶' may together form an alkylene group that may be interrupted by one or more divalent radicals that are not directly adjacent to one another and are selected from the group consisting of oxygen, imino,
where R⁷ is an alkyl radical that may be substituted by one or more radicals selected from the group consisting of alkoxy, hydroxy, nitro, carboxyl, carboxylic ester, carboxamide, sulfonate, sulfonic ester, sulfonamide, halogen and that may be interrupted by one or more divalent radicals that are not directly adjacent to one another and are selected from the group consisting of oxygen, imino,
where R⁸ is a p-valent organic bridging group that may be substituted by one or more radicals selected from the group consisting of alkyl, hydroxy, nitro, carboxyl, carboxylic ester, carboxamide, sulfonate, sulfonic ester, sulfonamide, halogen, and that may be interrupted by one or more divalent radicals that are not directly adjacent to one another and are selected from the group consisting of oxygen, imino,
X^{a-} is an organic anion, an inorganic anion or a mixture of these anions,
a denotes the valency of the anion X^{a-} and is an integer from 1 to 10 000,
p is an integer from 2 to 6,
m is an integer with the value of "p - 1",
n is an integer with the value of "1 / a",
o is an integer with the value of "(1 + m) / a",
**characterized in that**
(a) at least one compound of formula (III) or (IV) 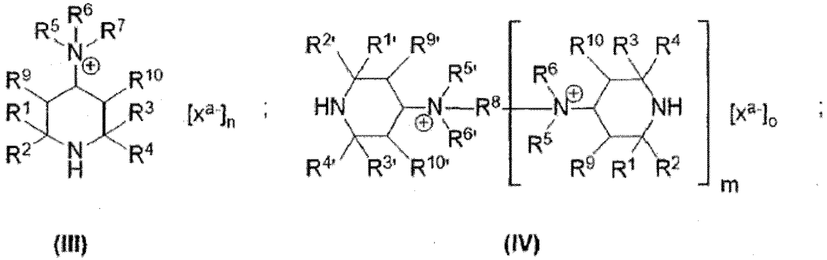 is reacted with H₂O₂ in a CO₂-containing, aqueous solution L₂ to form a compound of formula (I) or (II) and
(b) after the reaction according to step (a) H₂O₂, if this is then still present in the aqueous solution L₂, is removed from L₂, and optionally also CO₂ is partially removed from L₂,
whereby a CO₂-containing, aqueous solution L₃ comprising at least one compound of formula (I) or (II) is obtained,
(c) an acidic pH is established in L₃, whereby CO₂ is at least partially removed from L₃ and the aqueous electrolyte solution L₁ is obtained.

2. Process according to Claim 1,
where R¹, R², R³, R⁴, R¹', R²', R³', R⁴' are each independently a C₁-C₄ alkyl radical,
where R⁵, R⁶, R⁵', R⁶' are each independently selected from the group consisting of hydrogen, alkyl, where alkyl may be interrupted by one or more divalent radicals that are not directly adjacent to one another and are selected from the group consisting of oxygen, imino,
where in addition R⁵ and R⁶ may together form an alkylene group that may be interrupted by one or more divalent radicals that are not directly adjacent to one another and are selected from the group consisting of oxygen, imino,
where in addition R⁵' and R⁶' may together form an alkylene group that may be interrupted by one or more divalent radicals that are not directly adjacent to one another and are selected from the group consisting of oxygen, imino,
where R⁹, R¹⁰, R⁹', R¹⁰' are each independently selected from the group consisting of hydrogen, C₁-C₄ alkyl radical,
where R⁷ is C₁-C₆ alkyl, which may be interrupted by one or more divalent radicals that are not directly adjacent to one another and are selected from the group consisting of oxygen, imino,
where R⁸ is an alkylene group that may be substituted by one or more radicals selected from the group consisting of alkyl, hydroxy, nitro, carboxyl, carboxylic ester, carboxamide, sulfonate, sulfonic ester, sulfonamide, halogen and that may be interrupted by one or more divalent radicals that are not directly adjacent to one another and are selected from the group consisting of oxygen, imino,
X^{a-} is an inorganic anion,
a in compounds of formula (I) and (III) is an integer from 1 to 3 and in compounds of formula (II) and (IV) is an integer from 1 to 6,
m = 1,
n is an integer with the value of "1 / a",
o is an integer with the value of "2 / a".

3. Process according to Claim 2, where R¹, R², R³, R⁴, R¹', R²', R³', R⁴' are each methyl,
where R⁵, R⁶, R⁵', R⁶' are each independently selected from the group consisting of methyl, ethyl,
where R⁹, R¹⁰, R⁹', R¹⁰' are each hydrogen,
where R⁷ is selected from the group consisting of methyl, ethyl,
where R⁸ is a C₁-C₁₀ alkylene group,
X^{a-} is selected from the group consisting of halide ion with valency a = 1, hydroxy ion with valency a = 1, phosphate ion with valency a = 3, sulfate ion with valency a = 2, perchlorate ion with valency a = 1, hexafluorophosphate ion with valency a = 1, tetrafluoroborate ion with valency a = 1,
m = 1,
n is a number with the value of "1 / a",
o is a number with the value of "2 / a".

4. Process according to any of Claims 1 to 3 for preparing an aqueous solution L₁ of at least one compound of formula (I), where at least one compound of formula (III) is used and the definitions of the radicals R¹, R², R³, R⁴, R⁵, R⁶, R⁷, R⁹, R¹⁰, X^{a-} and of the variables n according to any of Claims 1 to 3 apply accordingly.

5. Process according to any of Claims 1 to 4, where step (a) is performed at a pH from 7.5 to < 10.5.

6. Process according to any of Claims 1 to 5, where step (a) is performed at a temperature from 20°C to 100°C and a pressure of 0.5 bar to 2 bar.

7. Process according to any of Claims 1 to 6, where before step (a) CO₂ in the form of dry ice and/or in gaseous form is dissolved in water, whereby a CO₂-containing, aqueous solution L₂ is obtained.

8. Process according to any of Claims 1 to 7, where the molar ratio of the molar amount of the CO₂ dissolved in the aqueous solution L₂ to the total molar amount of all compounds of formulae (III) and (IV) used in step (a) is in the range 10 : 1 to 1 : 1000.

9. Process according to any of Claims 1 to 8, where the molar ratio of the total weight of all compounds of formulae (III) and (IV) used in step (a) to the total weight of the H₂O₂ used in step (a) is in the range 9 : 1 to 1 : 9.

10. Process according to Claim 9, where the molar ratio of the total weight of all compounds of formulae (III) and (IV) used in step (a) to the total weight of the H₂O₂ used in step (a) is in the range 1 : 1.05 to 1 : 9.

11. Process according to Claim 10, where in step (b) H₂O₂ is removed by adjusting the aqueous solution L₂ to a pH of ≥ 10.5.

12. Process according to Claim 10, where in step (b) H₂O₂ is removed by reacting the aqueous solution L₂ in the presence of a catalyst.

13. Process according to any of Claims 1 to 12, where the proportion of all compounds of formula (I) and (II) in the aqueous electrolyte solution L₁ is in the range 10% to 80% by weight, based on the total weight of the aqueous electrolyte solution L₁.

## Revendications

1. Procédé de préparation d'une solution aqueuse d'électrolyte **L₁** comprenant au moins un composé de Formule **(I)** ou **(II)** 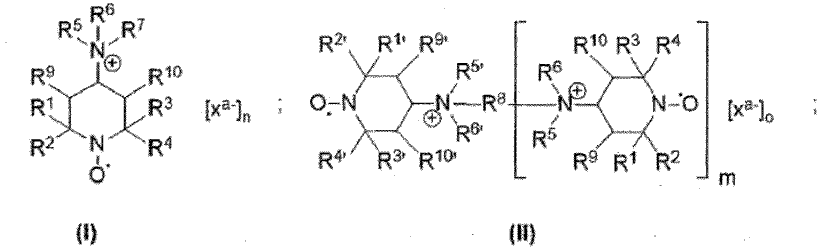
dans laquelle R¹, R², R³, R⁴, R¹', R²', R³', R⁴' représentent chacun indépendamment des autres un radical alkyle en C₁-C₆,
dans laquelle R⁹, R¹⁰, R⁹', R¹⁰' sont chacun indépendamment des autres choisis dans le groupe consistant en un hydrogène, un radical alkyle en C₁-C₆,
dans laquelle R⁵, R⁶, R⁵', R⁶' sont chacun indépendamment des autres choisis dans le groupe consistant en un hydrogène, un alkyle, un cycloalkyle, un aryle, un aralkyle, un hétérocyclyle, les radicaux alkyle, cycloalkyle, aryle, aralkyle, hétérocyclyle, pouvant être substitués par un ou plusieurs radicaux choisis dans le groupe consistant en les radicaux alcoxy, hydroxy, carboxyle, ester d'acide carboxylique, carboxamide, sulfonate, ester d'acide sulfonique, sulfonamide, halogène, par ailleurs l'alkyle pouvant être interrompu par un ou plusieurs radicaux divalents, non directement voisins l'un de l'autre, choisis dans le groupe consistant en un oxygène, un imino,
dans laquelle par ailleurs R⁵ et R⁶ peuvent former ensemble un groupe alkylène, qui peut être interrompu par un ou plusieurs radicaux divalents, non directement voisins l'un de l'autre, choisis dans le groupe consistant en un oxygène, un imino,
dans laquelle en outre R⁵' et R⁶' peuvent former ensemble un groupe alkylène, qui peut être interrompu par un ou plusieurs radicaux divalents, non directement voisins l'un de l'autre, choisis dans le groupe consistant en un oxygène, un imino,
dans laquelle R⁷ représente un radical alkyle, qui peut être substitué par un ou plusieurs radicaux choisis dans le groupe consistant en les radicaux alcoxy, hydroxy, nitro, carboxyle, ester d'acide carboxylique, carboxamide, sulfonate, ester d'acide sulfonique, sulfonamide, halogéno, et qui peut être interrompu par un ou plusieurs radicaux divalents, non directement voisins l'un de l'autre, choisis dans le groupe consistant en un oxygène, un imino,
dans laquelle R⁸ est un groupe pontant organique p-valant, qui peut être substitué par un ou plusieurs radicaux choisis dans le groupe consistant en les radicaux alkyle, hydroxy, nitro, carboxyle, ester d'acide carboxylique, carboxamide, sulfonate, ester d'acide sulfonique, sulfonamide, halogéno, et qui peut être interrompu par un ou plusieurs radicaux divalents, non directement voisins l'un de l'autre, choisis dans le groupe consistant en un oxygène, un imino,
X^{a-} représente un anion organique, un anion inorganique ou un mélange de ces anions,
a indique la valence des anions X^{a-} et représente un nombre entier de 1 à 10 000,
p représente un nombre entier de 2 à 6,
m représente un nombre entier de valeur « p - 1 »,
n représente un nombre de valeur « 1/a »,
o représente un nombre de valeur « (1 + m)/a »,
**caractérisé en ce que**
(a) au moins un composé de Formule **(III)** ou **(IV)** 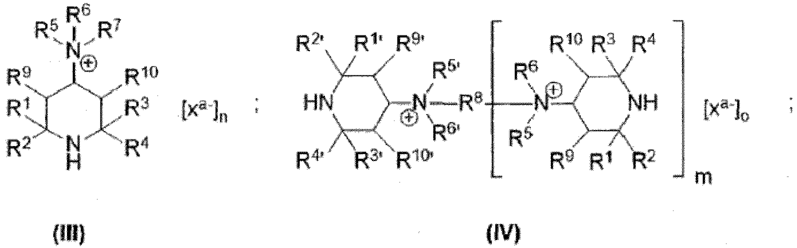 est mis à réagir avec H₂O₂ dans une solution aqueuse **L₂** contenant du CO₂, pour donner un composé de Formules respectivement **(I)** et **(II),** et
(b) après la réaction selon l'étape (a), le H₂O₂, si ce dernier est ensuite encore présent dans la solution aqueuse **L₂**, est éliminé de **L₂** et éventuellement aussi le CO₂ est partiellement éliminé de **L₂**,
ce qui permet d'obtenir une solution aqueuse **L₃** contenant du CO₂ comprenant au moins un composé de Formules respectivement **(I)** et **(II),**
(c) on ajuste un pH acide dans **L₃,** ce qui permet d'éliminer au moins partiellement le CO₂ de **L₃,** pour obtenir la solution aqueuse d'électrolyte **L₁.**

2. Procédé selon la revendication 1,
dans lequel R¹, R², R³, R⁴, R¹', R²', R³', R⁴' représentent chacun indépendamment des autres un radical alkyle en C₁-C₄,
dans lequel R⁵, R⁶, R⁵', R⁶' sont chacun indépendamment des autres choisis dans le groupe consistant en un hydrogène, un alkyle, l'alkyle pouvant être interrompu par un ou plusieurs radicaux divalents, non directement voisins l'un de l'autre, choisis dans le groupe consistant en un oxygène, un imino,
dans lequel par ailleurs R⁵ et R⁶ peuvent former ensemble un groupe alkylène, qui peut être interrompu par un ou plusieurs radicaux divalents, non voisins l'un de l'autre, choisis dans le groupe consistant en un oxygène, un imino,
dans lequel par ailleurs R⁵' et R⁶' peuvent former ensemble un groupe alkylène, qui peut être interrompu par un ou plusieurs radicaux divalents, non directement voisins l'un de l'autre, choisis dans le groupe consistant en un oxygène, un imino,
dans lequel R⁹, R¹⁰, R⁹', R¹⁰' sont chacun indépendamment des autres choisis dans le groupe consistant en un hydrogène, un radical alkyle en C₁-C₄,
dans lequel R⁷ représente un alkyle en C₁-C₆, qui peut être interrompu par un ou plusieurs radicaux divalents, non directement voisins l'un de l'autre, choisis dans le groupe consistant en un oxygène, un imino,
dans lequel R⁸ représente un groupe alkylène, qui peut être substitué par un ou plusieurs radicaux choisis dans le groupe consistant en les radicaux alkyle, hydroxy, nitro, carboxyle, ester d'acide carboxylique, carboxamide, sulfonate, ester d'acide sulfonique, sulfonamide, halogéno, et qui peut être interrompu par un ou plusieurs radicaux divalents, non directement voisins l'un de l'autre, choisis dans le groupe consistant en un oxygène, un imino,
X^{a-} représente un anion inorganique,
a, dans les composés de Formules **(I)** et **(III)**, représente un nombre entier de 1 à 3 et, dans les composés de Formules **(II)** et **(IV)**, un nombre entier de 1 à 6,
m = 1,
n représente un nombre de valeur « 1/a »,
o représente un nombre de valeur « 2/a ».

3. Procédé selon la revendication 2, dans lequel R¹, R², R³, R⁴, R¹', R²', R³', R⁴' représentent chacun un méthyle,
dans lequel R⁵, R⁶, R⁵', R⁶' sont chacun indépendamment des autres choisis dans le groupe consistant en un méthyle, un éthyle,
dans lequel R⁹, R¹⁰, R⁹', R¹⁰' représentent chacun un hydrogène,
dans lequel R⁷ est choisi dans le groupe consistant en un méthyle, un éthyle,
dans lequel R⁸ représente un groupe alkylène en C₁-C₁₀,
X^{a-} est choisi dans le groupe consistant en un ion halogénure de valence a = 1, un ion hydroxy de valence a = 1, un ion phosphate de valence a = 3, un ion sulfate de valence a = 2, un ion perchlorate de valence a = 1, un ion hexafluorophosphate de valence a = 1, un ion tétrafluoroborate de valence a = 1,
m = 1,
n représente un nombre de valeur « 1/a »,
o représente un nombre de valeur « 2/a ».

4. Procédé selon l'une des revendications 1 à 3 pour la fabrication d'une solution aqueuse **L₁** d'au moins un composé de Formule **(I),** dans lequel on utilise au moins un composé de Formule **(III),** et les définitions des radicaux R¹, R², R³, R⁴, R⁵, R⁶, R⁷, R⁹, R¹⁰, X^{a-} et des variables n correspondent à celles de l'une des revendications 1 à 3.

5. Procédé selon l'une des revendications 1 à 4, dans lequel l'étape (a) est mise en œuvre à un pH de 7,5 à < 10,5.

6. Procédé selon l'une des revendications 1 à 5, dans lequel l'étape (a) est mise en œuvre à une température de 20 °C à 100 °C et sous une pression de 0,5 bar à 2 bar.

7. Procédé selon l'une des revendications 1 à 6, dans lequel, avant l'étape (a), on dissout le CO₂ sous forme de neige carbonique et/ou sous forme gazeuse dans de l'eau, ce qui permet d'obtenir une solution aqueuse **L₂** contenant du CO₂.

8. Procédé selon l'une des revendications 1 à 7, dans lequel le rapport en moles entre la quantité de matière du CO₂ dissous dans la solution aqueuse **L₂** et la totalité de la quantité de matière de l'ensemble des composés utilisés dans l'étape (a), de Formules **(III)** et **(IV),** est compris dans la plage de 10:1 à 1:1 000.

9. Procédé selon l'une des revendications 1 à 8, dans lequel le rapport en moles entre le poids total de l'ensemble des composés utilisés dans l'étape (a) de Formules **(III)** et **(IV)** et le poids total du H₂O₂ utilisé dans l'étape (a) est compris dans la plage de 9:1 à 1:9.

10. Procédé selon la revendication 9, dans lequel le rapport en moles entre le poids total de l'ensemble des composés utilisés dans l'étape (a) de Formules **(III)** et **(IV)** et le poids total du H₂O₂ utilisé dans l'étape (a) est compris dans la plage de 1:1,05 à 1:9.

11. Procédé selon la revendication 10, dans lequel, dans l'étape (b), le H₂O₂ est éliminé par ajustement de la solution aqueuse **L₂** à un pH ≥ 10,5.

12. Procédé selon la revendication 10, dans lequel, dans l'étape (b), le H₂O₂ est éliminé par réaction de la solution aqueuse **L₂** en présence d'un catalyseur.

13. Procédé selon l'une des revendications 1 à 12, dans lequel la proportion de l'ensemble des composés de Formules **(I)** et **(II)** dans la solution aqueuse d'électrolyte **L₁** est comprise dans la plage de 10 à 80 % en poids par rapport au poids total de la solution aqueuse d'électrolyte **L₁**.
